# FASCICULE DE BREVET EUROPEEN

(11) **EP 2 652 512 B1**
(45) Date de publication et mention de la délivrance du brevet: **14.03.2018**
(21) Numéro de dépôt: 11805225.7
(22) Date de dépôt: 12.12.2011
(51) Int. Cl.: G01N 33/68, C07K 7/06, C07K 7/08, C12Q 1/34, C12N 9/64

(54) **UTILISATION DE L'IDE COMME BIOMARQUEUR D'UN ETAT DU CUIR CHEVELU**
VERWENDUNG VON IDE ALS BIOMARKER FÜR KOPFHAUTERKRANKUNGEN
USE OF IDE AS A BIOMARKER FOR A SCALP CONDITION

(30) Priorité: 13.12.2010 FR 1060429; 23.12.2010 US 201061457083 P
(43) Date de publication de la demande: 23.10.2013
(73) Titulaire: L'Oréal, 75008 Paris (FR)
(72) Inventeur: DELATTRE, Caroline, F-95470 Vemars (FR); SIRVEN, Philémon, F-78300 Poissy (FR); BERNARD, Dominique, F-92170 Vanves (FR)
(74) Mandataire: Nony
(86) Numéro de dépôt international: PCT/IB2011/055600
(87) Numéro de publication internationale: WO 2012/080929

(56) Documents cités:
- WO-A2-03/011895
- WO-A2-2005/040203
- WO-A2-2007/127290
- WO-A2-2008/021290
- WO-A2-2008/156701
- WO-A2-2010/037135
- FR-A1- 2 667 778
- US-A1- 2010 173 428
- US-A1- 2010 209 427
- DATABASE Geneseq [Online] 21 août 2008 (2008-08-21), "Psoriasis associated human protein SEQ ID NO: 20450.", XP002657498, extrait de EBI accession no. GSP:ARY79583 Database accession no. ARY79583 & WO 2008/016356 A2 (GENIZON BIOSCIENCES [CA]; BELOUCHI ABDELMAJID [CA]; RAELSON JOHN VERNE) 7 février 2008 (2008-02-07)
- CABROL CHRISTELLE ET AL: "Small-Molecule Activators of Insulin-Degrading Enzyme Discovered through High-Throughput Compound Screening", PLOS ONE, vol. 4, no. 4, avril 2009 (2009-04), XP002657499, ISSN: 1932-6203

## Description

La présente invention se rapporte au domaine des biomarqueurs et des cibles cosmétiques et/ou thérapeutiques de la peau, en particulier d'un état pelliculaire du cuir chevelu, ainsi qu'à leur mise en oeuvre à titre d'agents actifs.

Au sens de l'invention, on entend désigner par « peau » l'ensemble de l'épiderme d'un corps humain, y compris les muqueuses et les régions cutanées couvertes de poils ou de cheveux. Plus particulièrement, la peau considérée dans la présente invention est de préférence les lèvres, la peau du visage, du décolleté ou du cuir chevelu, et de manière plus préférée encore, la peau du cuir chevelu.

La peau est un tissu dont les cellules sont jointives, et solidaires les unes des autres. Il forme un revêtement externe comprenant des glandes sébacées ou sudoripares, et les follicules pileux. La peau, et notamment le cuir chevelu, sont des épithéliums à renouvellement continuel. Le renouvellement, ou desquamation, est un processus coordonné et finement régulé aboutissant à l'élimination des cellules superficielles, de façon insensible et non visible.

Toutefois, une desquamation anormale ou irrégulière des cellules du *stratum corneum,* pour diverses raisons, peut conduire à la formation d'amas de cellules de grandes tailles, épais, visibles à l'oeil nu, et dénommés « squames », ou « pellicules » dans le cadre du cuir chevelu, ou dans d'autres situations, à un amincissement du *stratum corneum.* Les troubles de la desquamation, résultant d'une desquamation anormale ou irrégulière, peuvent aboutir à une fragilité, voire un défaut des propriétés barrières de l'épiderme.

A titre d'exemple de facteurs favorisant l'apparition de squames ou de pellicules, on peut mentionner le stress, la période hivernale, un excès de sébum, un défaut d'hydratation, ou la colonisation de la peau ou des follicules pileux par la levure *Malassezia sp..* Ces facteurs ont, notamment, pour caractère commun de provoquer et/ou favoriser un état inflammatoire cutané. Une telle inflammation renforce l'apparition, voire augmente la présence de squames ou de pellicules. En particulier, les levures de type *Malassezia sp.,* constituant une partie de la flore commensale normale à la surface du cuir chevelu chez des sujets sans pellicule, voient leur proportion croître substantiellement en cas de pellicules, ou en cas de dermite séborrhéique associée. Le déséquilibre de l'écoflore du cuir chevelu est un facteur favorisant, voire renforçant la présence de pellicules.

La présence de squames ou les états pelliculaires peuvent être des états chroniques, fréquents, récidivants, et socialement invalidants du fait de leur caractère inesthétique manifeste. Qui plus est, les états pelliculaires du cuir chevelu ou la desquamation anormale de la peau peuvent se traduire par une altération de la fonction barrière de l'épiderme, ou générer des sensations de démangeaison ou prurit, conduisant à des comportements de grattage amplifiant le phénomène d'apparition de squames ou de pellicules, et, en retour, l'irritation du cuir chevelu ou de la peau.

Les états pelliculaires du cuir chevelu peuvent être de type gras ou de type sec. Les états pelliculaires secs du cuir chevelu se manifestent plus fréquemment, et sont amplifiés, lors de troubles de l'hydratation de la peau, et notamment lors de sécheresse importante de l'épiderme du cuir chevelu. Egalement, le cuir chevelu étant riche en glandes sébacées, un état pelliculaire peut se développer plus facilement en présence excessive de sébum et être plus facilement prurigineux. Ainsi, une sécrétion excessive de sébum, ou hyperséborrhée, favorise l'apparition d'un état pelliculaire gras du cuir chevelu, ou pellicules grasses, généralement associé à des désagréments, des sensations d'inconfort, des désordres esthétiques, voire une pathologie cutanée.

Les états pelliculaires répondent, en général, à différents traitements locaux ou systémiques. Cependant, l'efficacité de ces traitements n'est que suspensive et implique un suivi rigoureux de la part de l'utilisateur (fréquence d'utilisation et temps d'application suffisant). Or, un usage quotidien et à long terme de ces traitements peut entraîner un phénomène d'accoutumance réduisant leur efficacité, et généralement associée à un phénomène de rebond survenant à la cessation du traitement. Ce phénomène se manifeste par une hyperséborrhée, aggravant l'état pelliculaire et altérant la fonction barrière du cuir chevelu. Par ailleurs, l'agressivité de certains actifs antipelliculaires vis-à-vis des cellules épidermiques ou de l'écoflore du cuir chevelu peut également affecter les fonctions barrières de ce dernier et provoquer une aggravation de l'état pelliculaire. Enfin, l'efficacité des traitements antipelliculaires est souvent lente à se développer et nécessite une application rigoureuse sur le long terme. Ce temps de latence conduit fréquemment à un défaut du suivi du traitement. En conséquence, de nombreux échecs surviennent dans la mise en oeuvre de ces traitements.

De nombreux facteurs épidermiques, dont l'expression, l'activité biologique ou la maturation sont altérées, diminuées ou augmentées, sont connus pour être impliqués, directement ou indirectement, dans le processus de renouvellement, ou de desquamation de la peau, et notamment du cuir chevelu.

Ces facteurs peuvent être utilisés à titre de biomarqueurs de la peau, comme cibles de criblage, voire comme actifs cosmétiques.

Toutefois, il demeure encore de nombreuses inconnues quant au mécanisme intime et aux facteurs impliqués dans la desquamation de la peau, et en particulier dans l'apparition des pellicules.

Il existe donc un besoin de disposer de nouveaux biomarqueurs permettant de caractériser la desquamation de la peau, et plus particulièrement un état pelliculaire du cuir chevelu.

Il existe encore un besoin de disposer de nouvelles cibles pour le criblage d'agents actifs ou de traitements physiques pour le soin de la peau, en particulier pour prévenir et/ou traiter un trouble de la desquamation de la peau, et plus particulièrement un état pelliculaire du cuir chevelu.

Il existe encore un besoin de disposer de nouveaux actifs ou de nouveaux traitements pour prévenir et/ou traiter un trouble de la desquamation de la peau, et plus particulièrement un état pelliculaire du cuir chevelu.

Il existe encore un besoin de disposer de nouvelles cibles cosmétiques pour le soin de la peau, en particulier pour la prévention et/ou le traitement d'un trouble de la desquamation de la peau, et plus particulièrement un état pelliculaire du cuir chevelu.

Il demeure également un besoin de disposer de nouveaux traitements cosmétiques pour prévenir, réduire et/ou traiter les états pelliculaires du cuir chevelu qui soient efficaces et dépourvus d'effets secondaires susceptibles d'affecter une bonne observance.

Il existe encore un besoin de disposer d'un traitement des états pelliculaires du cuir chevelu n'affectant pas l'écoflore du cuir chevelu, voire renforçant la présence d'une écoflore saine.

Il existe également un besoin de disposer d'un traitement des états pelliculaires apte à maintenir, voire à renforcer, l'hydratation du cuir chevelu.

Il existe un besoin de disposer d'un traitement des états pelliculaires apte à maintenir, voire à renforcer, les propriétés barrières du cuir chevelu.

Il existe un besoin de disposer de traitements des états pelliculaires qui soient dépourvus des effets secondaires précités, et en particulier qui n'induisent pas d'hyperséborrhées, de dermites séborrhéiques ou d'états prurigineux.

Il existe également un besoin de disposer d'un traitement des états pelliculaires qui n'induise pas d'états inflammatoires.

La présente invention a pour objet de satisfaire à ces besoins.

Le présent texte décrit l'utilisation (i) d'au moins une séquence d'acides aminés codée par une séquence d'acides nucléiques représentée par SEQ ID NO: 1, un analogue ou un fragment de SEQ ID NO: 1, ou (ii) d'au moins de ladite séquence d'acides nucléiques, pour cribler des agents actifs ou des traitements physiques susceptibles de moduler l'activité, l'expression ou la maturation de ladite séquence d'acides aminés ou de ladite séquence d'acides nucléiques pour prévenir et/ou traiter une desquamation anormale de la peau, et de préférence un état pelliculaire du cuir chevelu.

De manière inattendue, au cours d'une étude de protéomique différentielle par approche électrophorèse 2D, les inventeurs ont observé à partir de prélèvements non-invasifs du cuir chevelu que l'Insulin Degrading Enzyme, ou Insulysine, ou Insulinase ou IDE, se révélait être un biomarqueur sensible et spécifique d'un état pelliculaire du cuir chevelu.

Plus précisément, les inventeurs ont observé que le taux d'expression de l'IDE, et plus particulièrement de peptides issus de l'IDE et identifiés par les séquences SEQ ID NO: 8 à SEQ ID NO: 14, était systématiquement augmenté dans les cuirs chevelus présentant un état pelliculaire par rapport aux cuirs chevelus non pelliculaires. C'est la première fois qu'est mise en relation une variation de l'expression de l'IDE et une desquamation anormale d'une peau. Ainsi, à la connaissance des inventeurs, cette enzyme n'a jamais été identifiée comme marqueur d'une desquamation anormale, et encore moins d'un état pelliculaire.

Cette observation valide la mise en oeuvre de l'IDE ou de fragments peptidiques issus de celle-ci, ou d'acides nucléiques codant pour ces polypeptides comme biomarqueur de la peau ou pour cribler de nouveaux agents actifs à l'égard de la desquamation anormale de la peau, et notamment des états pelliculaires, ainsi que l'utilisation d'agents modulateurs de l'activité de cette enzyme pour la prévention et/ou le traitement d'un état pelliculaire du cuir chevelu.

Au sens de l'invention on entend par « expression » à l'égard d'une séquence d'acides aminés, par exemple une protéine ou un peptide, ou d'une séquence d'acides nucléiques, par exemple un ARNm, sa teneur ou la variation de sa teneur par rapport à une référence.

Au sens de l'invention on entend par « maturation » à l'égard d'une séquence d'acides aminés, par exemple une protéine ou un peptide, ou d'une séquence d'acides nucléiques, par exemple un ARNm, les modifications qui suivent leur synthèse dans un environnement cellulaire. Par exemple, dans le cas d'une séquence d'acides aminés, on entend par « maturation », les modifications post-traductionnelles, telle la glycosylation, la farnésylation ou l'acétylation de certains acides aminés, ou les étapes protéolytiques conduisant à l'élimination de séquences dites « signal » ou « sécrétoire » ou à la libération de séquences présentant des propriétés biologiques particulières. Dans le cas d'une séquence d'acides nucléiques, on entend par « maturation », par exemple, l'épissage alternatif d'un pré-ARNm.

Au sens de l'invention on entend par « activité », à l'égard d'une séquence d'acides aminés, par exemple une protéine ou un peptide, l'activité biologique de la séquence d'acides aminés, le cas échéant après maturation, telle qu'une activité enzymatique, une activité d'agoniste ou d'antagoniste vis-à-vis d'un récepteur, ou d'activateur ou d'inhibiteur d'une enzyme, ou une activité dite de « structure ».

Plus particulièrement, au sens de l'invention, on entend par « activité » à l'égard d'une séquence d'acides aminés de l'invention représentée par SEQ ID NO 8, son activité protéolytique, notamment à l'égard de ses substrats habituels, tels que l'insuline, le glucagon, la bradykinine ou la kallidine.

Au sens de l'invention on entend par « activité », à l'égard d'une séquence d'acides nucléiques, par exemple un ARNm, sa traduction.

L'IDE, également dénommée Insulysine ou Insulinase, est une zinc-métalloprotéase (P14735 ; EC=3.4.24.56) de 1019 acides aminés et de 110kDa qui appartient à la famille des protéases M16A. Elle est impliquée dans l'hydrolyse de petits peptides bioactifs, tels que l'insuline, la β-amyloïde, l'amyline, le glucagon, l'« Insulin-Growth factor II » (IGF-II), la β-endorphine, la somatostatine, et l'atrial natriuretic peptide (Guo et al. 2009).

L'expression de l'IDE est particulièrement abondante dans le cerveau, le foie et les muscles et a, notamment, été localisée dans la couche granuleuse et est décrite comme présente dans le *stratum corneum* (Radulescu *et al.,* 2007). L'IDE a été particulièrement impliquée dans la dégradation des dépôts β-amyloïde caractéristiques de la maladie d'Alzheimer (Kim *et al.* 2007; Miners *et al.* 2009). Elle est aussi impliquée dans la pathologie du diabète de type II et l'hyperinsulinémie (Groves *et al.* 2003).

Elle est également décrite comme étant associée à des défauts de cicatrisation au niveau des épithéliums qui peuvent être compensés par apport d'insuline et par des inhibiteurs d'IDE (Shearer *et al.* 1997). L'IDE pourrait également être associée à une sensibilité virale (Ali *et al.* 2009).

Le présent texte décrit l'utilisation cosmétique d'une quantité efficace d'au moins un agent modulateur de l'activité, de l'expression ou de la maturation d'une séquence d'acides aminés de l'invention, ou d'une séquence d'acides nucléiques de l'invention, à titre d'agent actif pour prévenir et/ou traiter un trouble des propriétés barrières du cuir chevelu.

De préférence, un agent modulateur est un agent inhibiteur de l'activité enzymatique d'une séquence d'acides aminés de l'invention.

De manière préférée encore, un tel agent inhibiteur peut être mis en oeuvre pour prévenir et/ou traiter un état pelliculaire du cuir chevelu.

Au sens de la présente invention, on entend par « quantité efficace » d'un composé de l'invention, une quantité suffisante et nécessaire de ce composé pour obtenir un effet recherché, et plus particulièrement un effet cosmétique ou de soin à l'égard d'une desquamation anormale ou irrégulière de la peau, et de préférence d'un état pelliculaire du cuir chevelu.

Au sens de l'invention, on entend par « prévenir », le fait de réduire le risque de survenue ou de ralentir la survenue d'un phénomène donné, par exemple dans la présente invention, une desquamation anormale ou irrégulière de la peau, et de préférence d'un état pelliculaire du cuir chevelu.

Le présent texte décrit l'utilisation (i) d'au moins une séquence d'acides aminés de l'invention, ou (ii) d'au moins une séquence d'acides nucléiques de l'invention, à titre de biomarqueur de la desquamation de la peau, et de préférence d'un état pelliculaire du cuir chevelu.

Selon un premier objet, la présente invention concerne l'utilisation *in vitro* ou *ex vivo* (i) d'au moins une séquence d'acides aminés codée par une séquence d'acides nucléiques SEQ ID NO: 1, un analogue ou un fragment de SEQ ID NO: 1, ledit analogue ayant une identité de séquence d'au moins 85 % avec la séquence SEQ ID NO : 1 et codant pour une séquence d'acides aminés ayant une activité biologique de même nature que la séquence d'acides aminés codée par la séquence SEQ ID NO : 1, ledit fragment comprenant de 9 à 300 paires de bases consécutives de la séquence SEQ ID NO : 1 et codant pour une séquence d'acides aminés ayant une activité biologique de même nature que la séquence d'acides aminés codée par la séquence SEQ ID NO : 1, ou (ii) d'au moins de ladite séquence d'acides nucléiques, à titre de biomarqueur d'un état pelliculaire du cuir chevelu.

Au sens de l'invention, on entend par « biomarqueur », une molécule ou l'activité d'une molécule, dont la présence ou l'absence, la teneur ou le degré d'activité, ou une variation de ces paramètres est caractéristique d'un processus biologique, physiologique ou pathologique, ou de l'impact ou de l'effet induit par l'administration d'un agent actif ou d'un traitement physique sur un tel processus.

Le présent texte décrit également l'utilisation (i) d'au moins une séquence d'acides aminés de l'invention, ou (ii) d'au moins une séquence d'acides nucléiques de l'invention, pour caractériser l'efficacité d'un traitement cosmétique de la peau.

Selon un mode de réalisation préféré, un traitement cosmétique dont l'efficacité est caractérisée peut être un traitement cosmétique d'une desquamation anormale de la peau, et de préférence d'un état pelliculaire du cuir chevelu.

Selon un autre de ses objets, la présente invention concerne l'utilisation (i) d'au moins une séquence d'acides aminés de l'invention, ou (ii) d'au moins une séquence d'acides nucléiques de l'invention, pour caractériser l'efficacité d'un traitement cosmétique d'un état pelliculaire du cuir chevelu.

Le présent texte décrit également l'utilisation (i) d'au moins une séquence d'acides aminés de l'invention, ou (ii) d'au moins une séquence d'acides nucléiques de l'invention, pour sélectionner parmi un ensemble d'agents actifs connus pour prévenir et/ou traiter une desquamation anormale de la peau, et de préférence un état pelliculaire du cuir chevelu, un agent actif présumé exercer un effet bénéfique maximal à l'égard de ladite desquamation ou dudit état pelliculaire.

Selon un autre mode de réalisation, la présente invention concerne l'utilisation (i) d'au moins une séquence d'acides aminés de l'invention, ou (ii) d'au moins une séquence d'acides nucléiques de l'invention, pour sélectionner parmi un ensemble d'agents actifs connus pour prévenir et/ou traiter un état pelliculaire du cuir chevelu, un agent actif présumé exercer un effet bénéfique maximal à l'égard dudit état pelliculaire.

Le présent texte décrit en outre l'utilisation d'une quantité efficace (i) d'au moins une séquence d'acides aminés de l'invention, ou (ii) d'au moins une séquence d'acides nucléiques de l'invention, ou (iii) d'au moins un agent modulateur de l'invention, pour préparer une peau reconstruite isolée.

Avantageusement, ce modèles de peau isolée peut être mis en oeuvre pour reproduire un trouble de la desquamation de la peau, et notamment d'un état pelliculaire du cuir chevelu.

Le présent texte décrit aussi un procédé pour caractériser une desquamation anormale de la peau, et de préférence un état pelliculaire du cuir chevelu, comprenant au moins les étapes consistant à :
a) effectuer, dans un échantillon isolé d'une peau, et de préférence d'un cuir chevelu, une mesure qualitative ou quantitative de l'expression, de la maturation ou de l'activité de ladite séquence d'acides aminés ou de ladite séquence d'acides nucléiques, et
b) comparer ladite mesure effectuée à l'étape a) à une mesure de référence.

Selon encore un autre de ses objets, la présente invention concerne un procédé in vitro ou ex vivo pour caractériser un état pelliculaire du cuir chevelu comprenant au moins les étapes consistant à :
a) effectuer, dans un échantillon isolé d'un cuir chevelu, une mesure de l'expression, de la maturation ou de l'activité d'une séquence d'acides aminés de l'invention, et
b) comparer ladite mesure effectuée à l'étape a) à une mesure de référence ;
une augmentation de l'activité, de l'expression ou de la maturation de ladite séquence d'acides aminés étant indicative d'un état pelliculaire du cuir chevelu.

Le présent texte décrit un procédé de criblage d'agents actifs ou de traitements physiques susceptibles de moduler l'activité, l'expression ou la maturation d'une séquence d'acides aminés de l'invention ou d'une séquence d'acides nucléiques de l'invention, pour prévenir et/ou traiter une desquamation anormale de la peau, et de préférence un état pelliculaire du cuir chevelu, comprenant au moins les étapes consistant à :
a) disposer ladite séquence d'acides aminés ou ladite séquence d'acides nucléiques dans des conditions propices à l'activité, l'expression ou la maturation desdites séquences,
b) mettre en contact ladite séquence d'acides aminés ou ladite séquence d'acides nucléiques avec au moins un agent actif à tester, ou exposer ladite séquence d'acides aminés ou ladite séquence d'acides nucléiques avec un traitement physique à tester,
c) effectuer une mesure qualitative ou quantitative de l'expression, de la maturation ou de l'activité de ladite séquence d'acides aminés ou de ladite séquence d'acides nucléiques, et
d) comparer ladite mesure à une mesure de référence.

Le présent texte décrit également un procédé cosmétique pour prévenir et/ou traiter une desquamation anormale de la peau, et de préférence un état pelliculaire du cuir chevelu, chez un individu en ayant besoin, comprenant au moins une étape consistant à administrer audit individu, au moins une composition comprenant à titre d'agent actif au moins un agent modulateur de l'activité, de l'expression ou de la maturation desdites séquences, de préférence comme défini ci-après.

Le présent texte décrit en outre un procédé cosmétique pour caractériser l'efficacité d'un traitement cosmétique d'une desquamation anormale de la peau, et de préférence d'un état pelliculaire du cuir chevelu, chez un individu en ayant besoin, comprenant au moins les étapes consistant à :
a) effectuer, avant la mise en oeuvre du traitement cosmétique, dans un premier échantillon de peau isolée, de préférence de cuir chevelu isolé, prélevé chez ledit individu, au moins une première mesure qualitative ou quantitative de l'expression, de la maturation ou de l'activité d'au moins une séquence d'acides aminés de l'invention, ou d'au moins une séquence d'acides nucléiques de l'invention,
b) effectuer, après la mise en oeuvre du traitement cosmétique, dans un second échantillon de peau isolée, de préférence de cuir chevelu isolé, prélevé chez ledit individu, au moins une seconde mesure, qualitative ou quantitative de l'expression, de la maturation ou de l'activité de ladite séquence d'acides aminés ou de ladite séquence d'acides nucléiques, et
c) comparer les première et deuxième mesures, notamment afin d'en déduire une information relative à un effet au moins de la mise en oeuvre du traitement cosmétique.

Selon encore un autre de ces objets, la présente invention concerne un procédé cosmétique pour caractériser l'efficacité d'un traitement cosmétique d'un état pelliculaire du cuir chevelu chez un individu en ayant besoin, comprenant au moins les étapes consistant à :
a) effectuer, avant la mise en oeuvre du traitement cosmétique, dans un premier échantillon de cuir chevelu isolé prélevé chez ledit individu, au moins une première mesure de l'expression, de la maturation ou de l'activité d'au moins une séquence d'acides aminés de l'invention, ou d'au moins une séquence d'acides nucléiques de l'invention,
b) effectuer, après la mise en oeuvre du traitement cosmétique, dans un second échantillon de cuir chevelu isolé prélevé chez ledit individu, au moins une seconde mesure, qualitative ou quantitative de l'expression, de la maturation ou de l'activité de ladite séquence d'acides aminés ou de ladite séquence d'acides nucléiques, et
c) comparer les première et deuxième mesures, notamment afin d'en déduire une information relative à un effet au moins de la mise en oeuvre du traitement cosmétique.

Selon un mode préféré de réalisation, un procédé ou une utilisation conforme à l'invention peuvent être mis en oeuvre *in vitro* ou *ex vivo.*

Le présent texte décrit un peptide isolé représenté par une séquence d'acides aminés choisie parmi SEQ ID NO: 9 à SEQ ID NO: 14, un analogue ou un fragment de celle-ci.

Selon encore un autre mode de réalisation, la présente invention concerne un peptide isolé choisi parmi SEQ ID NO : 9 à SEQ ID NO : 14, ou un analogue ayant une identité de séquence d'au moins 85% avec ledit peptide isolé et ayant une activité biologique de même nature que ledit peptide isolé.

Le présent texte décrit également une composition comprenant un peptide représenté par une séquence d'acides aminés choisie par SEQ ID NO: 8 à SEQ ID NO: 14, un analogue ou un fragment de celle-ci, ou une séquence d'acide nucléique codant pour un tel peptide.

Selon encore un autre de ces objets, la présente invention concerne une composition comprenant un peptide choisi parmi les SEQ ID NO: 9 à SEQ ID NO: 14 ou un analogue ayant une identité de séquence d'au moins 85% avec ledit peptide isolé et ayant une activité biologique de même nature que ledit peptide isolé ou une séquence d'acide nucléique codant pour un tel peptide.

Le présent texte décrit aussi un modèle pluricellulaire de peau comprenant au moins une cellule dans laquelle l'expression d'une protéine représentée par SEQ ID NO: 8, un fragment ou un analogue de celle-ci est réprimée ou augmentée. De préférence, un tel modèle cellulaire est un modèle *in vitro* ou *ex vivo.*

La présente invention a pour avantage de proposer un nouveau biomarqueur sensible et spécifique de la peau, et en particulier de la desquamation, notamment anormale, de la peau, et plus particulièrement d'un état pelliculaire du cuir chevelu.

L'observation de la présence de l'IDE dans le *stratum corneum,* et plus particulièrement de peptides issus spécifiquement de cette protéine, rend avantageusement possible une détermination quantitative ou qualitative de l'expression ou de l'activité de cette protéine, ou des peptides correspondant, par simple prélèvement topique.

La méthode de prélèvement peut par exemple être une technique de type corneodisque ou stripping consistant à appliquer sur l'épiderme considéré un élément adhésif. En décollant cet élément adhésif, une fraction de la surface de la peau est prélevée. Après extraction protéique, celle-ci peut être ensuite analysée par des méthodes conventionnelles, telles que le dosage immuno-enzymatique ELISA, ou une analyse Western-Blot, ou plus particulièrement par la méthode de protéomique différentielle par électrophorèse 2D.

Egalement, la présente invention a pour avantage de pouvoir mettre à disposition un nouveau biomarqueur convenant au criblage de nouveaux agents actifs ou de nouveaux traitements physiques pour prévenir et/ou traiter un trouble de la desquamation de la peau, et notamment un état pelliculaire du cuir chevelu.

Egalement, selon un autre avantage, la présente invention permet de proposer de nouveaux agents actifs pour prévenir et/ou traiter un trouble de la desquamation de la peau, et notamment un état pelliculaire du cuir chevelu.

Avantageusement, les nouveaux actifs proposés par la présente invention permettent de renforcer les propriétés barrières du cuir chevelu.

La protection et le renforcement des propriétés barrières du cuir chevelu permettent une diminution des états inflammatoires de la peau, le maintien d'une barrière équilibrée, de son intégrité et la conservation d'une écoflore équilibrée.

Le cuir chevelu est alors moins irrité et prurigineux, moins fragile et plus hydraté, et les états pelliculaires sont réduits.

### Séquences d'acides aminés et d'acides nucléiques

L'Insulin-Degrading Enzyme (IDE) ou Insulysine est une protéine de 1019 acides aminés (SEQ ID NO: 8) comportant une méthionine d'initiation qui est éliminée, et dont le gène est localisé sur le chromosome 10, locus 10q23-q25.

Sauf indication contraire, le terme « IDE » vise à désigner dans la présente demande les séquences d'acides aminés représentées par SEQ ID NO: 8, à SEQ ID NO: 14, ayant ou non subi une maturation post-traductionnelle.

Selon un mode de réalisation préféré, on entend désigner par IDE plus particulièrement la séquence d'acides aminés représentée par SEQ ID NO: 8, et de préférence encore la séquence d'acide aminé représentée par une séquence issue de SEQ ID NO: 8 dans laquelle la méthionine d'initiation a été éliminée.

Selon un mode de réalisation, une séquence d'acides aminés convenant à l'invention peut être codée par une séquence d'acides nucléiques représentées par SEQ ID NO: 1, ou un analogue ou un fragment de cette séquence.

Par « analogue » d'une séquence d'acides aminés ou d'une séquence d'acides nucléiques conformes à l'invention, on entend désigner toute séquence d'acides aminés ou d'acides nucléiques ayant une identité de séquence d'au moins 85 %, de préférence d'au moins 90 %, et plus préférentiellement d'au moins 95 % avec ladite séquence de référence, et, selon le cas, ayant une activité biologique de même nature ou codant pour une séquence d'acides aminés ayant une activité biologique de même nature. A titre d'analogue convenant à l'invention, on peut citer les séquences homologues identifiées dans la base Homologene (http://www.ncbi.nlm.nih.gov/homologene). Par « analogue d'une séquence d'acides nucléiques », on entend en particulier désigner une séquence d'acides nucléiques résultant de la dégénérescence du code des acides nucléiques, et codant pour une séquence d'acides aminés conforme à l'invention, notamment tel que défini précédemment.

Par « activité biologique de même nature » à l'égard d'une séquence d'acides aminés selon l'invention, on entend notamment les propriétés protéolytiques habituellement attribuées à l'IDE, telles que par exemple la capacité à hydrolyser l'insuline, la bradykinine, la kallidine, le peptide β-amyloïde ou le glucagon. Notamment, une séquence d'acides aminés selon l'invention est plus particulièrement caractérisée par sa capacité à dégrader des substrats formant des structures de type amyloïdes.

L'identité de séquence peut être déterminée par comparaison visuelle ou au moyen de tout outil informatique généralement utilisé dans le domaine, tel que les programmes BLAST disponibles sur www.ncbi.nlm.nih.gov et utilisés avec les paramètres configurés par défaut.

Un analogue d'une séquence d'acides aminés conforme à l'invention peut être un agent peptidomimétique.

Un analogue d'une séquence d'acides aminés de l'invention peut résulter de modifications issues d'une ou plusieurs mutation(s) et/ou de variation(s) dans les séquences des peptides selon l'invention provenant soit de la délétion ou de l'insertion d'un ou plusieurs acides aminés, soit de la substitution d'un ou plusieurs acides aminés, soit encore d'un épissage alternatif. Plusieurs de ces modifications peuvent être combinées.

Avantageusement, un analogue d'une séquence d'acides aminés de l'invention peut comprendre des substitutions conservatrices par rapport à cette séquence d'acides aminés de référence.

A titre d'exemple de mutations que l'on peut considérer dans la présente invention, ou peut mentionner, de manière non exhaustive, le remplacement d'un ou plusieurs résidus d'acides aminés par des résidus d'acides aminés ayant un indice hydropathique similaire sans pour autant affecter de manière sensible les propriétés biologiques du polypeptide. L'indice hydropathique est un indice attribué aux acides aminés en fonction de leur hydrophobicité et de leur charge (Kyte *et al.* 1982).

Une séquence d'acides aminés ou un analogue de celle-ci visé par la présente invention peut être une séquence d'acides aminés ayant subi une ou plusieurs maturation(s) post-traductionnelle(s).

Par « maturation(s) post-traductionnelle(s) », on entend englober l'ensemble des modifications qu'une séquence d'acides aminés est susceptible de subir à l'issue de sa synthèse dans une cellule, telle que par exemple une ou des phosphorylation(s), une ou des thiolation(s), une ou des acétylation(s), une ou des glycosylation(s), une ou des lipidation(s), telles qu'une farnésylation ou une palmitoylation, un réarrangement structural de type formation de ponts disulfures et/ou de type clivage à l'intérieur de la séquence peptidique.

Un analogue d'une séquence d'acides aminés présente, par ailleurs, sensiblement la même activité biologique que cette séquence d'acides aminés.

Il est par ailleurs connu qu'une séquence primaire d'acides aminés, peut comprendre des sites spécifiquement reconnus par des enzymes de type protéase, telle que la trypsine, qui, une fois la reconnaissance de ces sites effectuée vont induire le clivage de la séquence par protéolyse. Cette protéolyse résulte en la génération de divers peptides, ou fragments de séquences d'acides aminés de l'invention.

En conséquence, le présent texte décrit également des fragments de l'IDE, issus le cas échéant de sa protéolyse.

Au sens de l'invention, on entend par « fragment d'une séquence d'acides aminés » toute portion de la séquence d'acides aminés conforme à l'invention comprenant au moins 3, voire au moins 4, et mieux encore au moins 6 acides aminés consécutifs de ladite séquence de référence, ou comprenant de 3 à 100 acides aminés consécutifs de ladite séquence de référence, de préférence de 4 à 90, de préférence 6 à 80, et plus préférentiellement de 9 à 70 acides aminés consécutifs de ladite séquence, et a une activité biologique de même nature.

Au sens de la présente invention, on entend par « fragment d'une séquence d'acides nucléiques », une séquence d'acides nucléiques comprenant au moins 9, voire au moins 12, et mieux encore au moins 18 paires de bases consécutives de ladite séquence de référence, ou comprenant de 9 à 300 paires de bases consécutives de ladite séquence de référence, de préférence de 12 à 270, de préférence 18 à 240, et plus préférentiellement de 27 à 210 paires de bases consécutives de ladite séquence, et codant pour une séquence d'acides aminés ayant une activité biologique de même nature que la séquence d'acides aminés codée par ladite séquence.

Selon un mode de réalisation, une séquence d'acides aminés convenant à l'invention peut être une séquence d'acides aminés représentée par une séquence choisie parmi SEQ ID NO: 8 à 14, un analogue, ou un fragment de celle-ci et de préférence parmi SEQ ID NO: 9, SEQ ID NO: 10, SEQ ID NO: 11, SEQ ID NO: 12, SEQ ID NO: 13 et SEQ ID NO: 14, un analogue, ou un fragment de celle-ci.

Selon un autre mode de réalisation, une séquence d'acides aminés convenant à l'invention peut être naturelle ou synthétique, le cas échéant susceptible d'être obtenue après lyse enzymatique ou chimique de l'IDE ou par synthèse chimique ou biologique ou par extraction à partir d'un tissu biologique, comme par exemple la peau, exprimant naturellement cette séquence d'acides aminés ou après transfection de celui-ci, ainsi que les différentes formes post-traductionnelles de celui-ci, ou encore toute séquence d'acides aminés naturelle ou synthétique dont la séquence comprend totalement ou partiellement une séquence d'acides aminés précitée, par exemple les variants et les analogues.

L'homme de l'art peut obtenir une séquence d'acides aminés conforme à l'invention au moyen de procédés à base d'ADN recombinant, comme par exemple, ceux décrits dans le manuel « Molecular Cloning - A Laboratory Manual » (2ème édition), Sambrook et al., 1989, Vol. I-III, Coldspring Harbor Laboratory, Coldspring Harbor Press, NY, (Sambrook).

Selon un autre mode de réalisation, une séquence d'acides aminés convenant à l'invention peut également être une séquence d'acides aminés telle que définie précédemment, fusionnée avec une autre séquence d'acides aminés, un agent de ciblage hydrophile ou hydrophobe, un précurseur de bioconversion, un agent de marquage luminescent, radioactif ou colorimétrique, ou encore un agent de marquage pour anticorps.

De manière non limitative, on peut citer comme exemple de composés susceptibles d'être couplés à une séquence d'acides aminés conforme à l'invention des protéines fluorescentes comme la « Green Fluorescent Protein », des composés chimiques fluorescents, tels que la rhodamine, la fluorescéine, ou le Texas Rode®, des composés phosphorescents, des éléments radioactifs, tels que ³_{H}, ¹⁴C, ³⁵S, ¹²¹I ou ¹²⁵I, ou des agents de marquage colorimétrique comme les substrats chromogènes sensibles à l'action de la galactosidase, de la peroxydase, de la chloramphénicol acétyltransférase, de la luciférase ou de la phosphatase alcaline, ou un agent de marquage pour anticorps, tel que His-Tag.

Selon la nature des composés susceptibles d'être couplés avec une séquence d'acides aminés de l'invention, le couplage peut être opéré par des procédés chimiques, notamment au moyen de fonctions chimiques réactives ou par des procédés de biologie moléculaire connus de l'homme de l'art.

Selon un mode de réalisation, la présente invention concerne également des séquences d'acides nucléiques codant pour une séquence d'acides aminés de l'invention, et leur mise en oeuvre dans les différentes utilisations et procédés de l'invention.

Le présent texte décrit également à une séquence d'acides nucléiques, notamment d'acides désoxyribonucléiques, ou d'acides ribonucléiques, représentée par SEQ ID NO: 1, un analogue ou un fragment de celle-ci.

Ainsi, le présent test décrit également à une séquence d'acides nucléiques, notamment d'acides désoxyribonucléiques, ou d'acides ribonucléiques, représentée par SEQ ID NO: 1 ou un analogue de celle-ci tel que précédemment défini.

Avantageusement, une séquence d'acides aminés peut être codée par une séquence d'acides nucléiques choisie parmi une séquence représentée par SEQ ID NO: 1, SEQ ID NO: 2, SEQ ID NO: 3, SEQ ID NO: 4, SEQ ID NO: 5, SEQ ID NO: 6, ou SEQ ID NO: 7, un analogue ou un fragment de celle-ci.

De manière préférée, une séquence d'acides nucléiques de l'invention peut être représentée par une séquence choisie parmi SEQ ID NO: 2, SEQ ID NO: 3, SEQ ID NO: 4, SEQ ID NO: 5, SEQ ID NO: 6, ou SEQ ID NO: 7, un analogue ou un fragment de celle-ci.

Une séquence d'acides nucléiques de l'invention peut être de toutes origines possibles, à savoir soit animale, en particulier de mammifères et encore plus particulièrement humaine, soit végétale, soit de micro-organismes, tels que par exemple virus, phages, ou bactéries entre autres, ou encore de champignons, sans préjuger du fait qu'elles soient présentes de manière naturelle ou non dans ledit organisme d'origine.

Selon un mode de réalisation, l'invention se rapporte également aux séquences d'acides nucléiques isolées et purifiées codant pour une séquence d'acides aminés considérés selon l'invention, ainsi qu'à leurs analogues et fragments.

Une séquence d'acides nucléiques conforme à l'invention peut comprendre une séquence sens, anti-sens ou interférentielle correspondant à une séquence codant pour un polypeptide conforme à l'invention.

Le présent texte décrit également des séquences d'acides nucléiques, notamment d'acides ribonucléiques ou désoxyribonucléiques, comprenant une séquence sens ou anti-sens, notamment « small interferent RNA » (siRNA), correspondant au moins à une séquence codant pour une protéine ou un peptide de l'invention ou à une séquence d'acides nucléiques de l'invention, un analogue ou un fragment de celle-ci.

Le présent texte décrit également des séquences RNA ou DNA aptamères capables de moduler l'activité de l'enzyme.

Le texte décrit également des anticorps polyclonaux ou monoclonaux obtenus par des technologies classiques, ou de préférence des anticorps recombinants humains sélectionnés contre l'IDE par des technologies de phage display, telles que celles proposées par la société AbD Serotec, ou des « Nanobodies », tels que proposés par la société Ablynx.

### États pelliculaires et desquamation de la peau

Comme signifié précédemment, un cuir chevelu présentant une sécheresse excessive ou une sécrétion excessive de sébum, peut manifester un état pelliculaire, qui, selon le cas, peut se caractériser par la présence de pellicules sèches ou grasses, voire un prurit et/ou une inflammation de l'épiderme.

Les états pelliculaires secs traduisent une xérose du cuir chevelu, le cas échéant associée à un renouvellement excessivement rapide de son *stratum corneum.* Les pellicules sèches sont généralement de petites tailles, blanches ou grises, et se répartissent sur le cuir chevelu et les vêtements générant un effet visuel inesthétique. Les démangeaisons associées à la sécheresse du cuir chevelu peuvent conduire à un érythème, un prurit, voire un état inflammatoire.

Les états pelliculaires gras sont une des formes de dermites séborrhéiques. Les sujets qui en sont atteints ont un cuir chevelu érythémateux couvert par des squames larges, grasses et jaunes qui s'accumulent pour former des paquets. Ils ont un scalp prurigineux, et ont souvent des sensations de brulure sur les zones atteintes. Les états pelliculaires gras du cuir chevelu se manifestent, et sont amplifiés, lors d'une sécrétion excessive de sébum au niveau de l'épiderme du cuir chevelu. Les états pelliculaires gras dans leurs formes sévères peuvent être des formes de dermites séborrhéiques.

Ces phénomènes peuvent être amplifiés par la présence de microorganismes pathogènes, notamment *Malassezia sp..* Ces microorganismes dotés de la propriété de libérer des acides gras à partir du sébum peuvent altérer la fonction barrière de l'épiderme et générer des états inflammatoires.

Lors des états pelliculaires du cuir chevelu, la barrière cutanée est déséquilibrée, son intégrité et son hydratation sont altérées et son écoflore perturbée. La peau du cuir chevelu est irritée et prurigineuse, fragile, moins hydratée et sensible aux infections.

La mise en oeuvre d'une séquence d'acides aminés ou d'une séquence d'acide nucléiques de l'invention ou d'un agent modulateur de l'invention conduit à rétablir l'hydratation et l'écoflore et à diminuer le prurit du cuir chevelu. Cette diminution se traduit par une réduction des phases de grattage du scalp et de l'altération de la fonction barrière en résultant. L'efficacité du traitement est ainsi nettement améliorée et se développe de manière beaucoup plus rapide. La peau est alors moins irritée et moins prurigineuse et la présence des pellicules est réduite, voire éliminée.

Les utilisations, procédés et compositions selon l'invention, s'avèrent ainsi tout particulièrement efficaces :
- pour prévenir et/ou traiter les désordres, notamment esthétiques, du cuir chevelu associés à une sécheresse excessive, voire une xérose,
- pour prévenir et/ou traiter les désordres, notamment esthétiques, du cuir chevelu associés à un excès d'excrétion et/ou de sécrétion de sébum,
- pour prévenir et/ou traiter les états pelliculaires, qu'ils soient secs ou gras, du cuir chevelu,
- pour améliorer et/ou rétablir les défenses antimicrobiennes du cuir chevelu sec ou gras,
- pour améliorer le confort des peaux et cuirs chevelus,
- pour améliorer l'hygiène et/ou le soin du cuir chevelu,
- pour conférer une sensation de bien être au cuir chevelu,
- pour préserver et/ou renforcer l'intégrité des fonctions barrières de la peau du cuir chevelu,
- pour maintenir et/ou restaurer les propriétés biomécaniques du cuir chevelu,
- pour rétablir une écoflore équilibrée du cuir chevelu,
- pour prévenir et/ou traiter les prurits et/ou dermites séborrhéiques associés aux états pelliculaires du cuir chevelu, et/ou
- pour prévenir et/ou traiter les états inflammatoires associés aux états pelliculaires du cuir chevelu.

Avantageusement, l'invention peut être mise en oeuvre pour prévenir et/ou traiter un trouble des propriétés barrières du cuir chevelu.

Comme expliqué ci-après, les états pelliculaires du cuir chevelu considérés par l'invention sont distincts du psoriasis du cuir chevelu.

De manière plus générale, la présente invention peut être mise en oeuvre à l'égard de la desquamation, notamment anormale ou irrégulière, de la peau, en vue de maintenir ou restaurer l'homéostasie de la peau, et en particulier les propriétés barrières de l'épiderme. Ainsi, la présente invention peut également être mise en oeuvre pour prévenir et/ou traiter un trouble des propriétés barrières du cuir chevelu.

Selon la sévérité de la dérégulation de la desquamation, les troubles de la desquamation de la peau subséquents peuvent relever soit du domaine cosmétique, soit du domaine thérapeutique. Il relève des connaissances générales, et de la pratique habituelle, de l'homme de l'art de distinguer les troubles de la desquamation de la peau en fonction du domaine duquel il relève.

Une desquamation anormale ou irrégulière peut se traduire par un épaississement ou un amincissement du *stratum corneum,* parfois accompagné de désordres de la fonction barrière de la peau. Sur le plan esthétique, une peau présentant une desquamation anormale peut être une peau présentant des signes de sécheresse ou de xérose cutané, de rugosité, des pellicules, ou des squames.

Lorsque la desquamation anormale est fortement aggravée, l'état de la peau peut relever du domaine thérapeutique et présenter une dermatite atopique, une ichtyose, ou un psoriasis.

### Biomarqueur

Un biomarqueur de l'invention permet avantageusement, de caractériser la desquamation, notamment anormale, de la peau, et de préférence un état pelliculaire du cuir chevelu.

Selon un mode de réalisation de l'invention, une augmentation ou une diminution de l'activité, de l'expression ou de la maturation d'un biomarqueur de l'invention peut être indicative d'une desquamation anormale ou irrégulière de la peau, et de préférence d'un état pelliculaire du cuir chevelu.

Selon un mode de réalisation préféré, une augmentation de l'activité, de l'expression ou de la maturation d'un biomarqueur de l'invention peut être indicative d'un état pelliculaire du cuir chevelu.

Un biomarqueur peut être mis en oeuvre pour caractériser l'efficacité d'un traitement cosmétique de la peau, et notamment à l'égard d'une desquamation anormale ou irrégulière de la peau. En particulier, le traitement cosmétique dont l'efficacité est caractérisée peut être un traitement d'un état pelliculaire du cuir chevelu.

Une augmentation ou une diminution de l'activité, de l'expression ou de la maturation du biomarqueur peut être indicative d'un traitement cosmétique efficace, et notamment pour exercer un effet bénéfique sur une desquamation anormale ou irrégulière de la peau, et en particulier à l'égard d'un état pelliculaire du cuir chevelu.

Une diminution ou une augmentation de l'activité, de l'expression ou de la maturation du biomarqueur peut être déterminée par comparaison à une mesure de référence obtenue selon toute méthode connue de l'homme de l'art.

Une « mesure de référence » au regard d'un paramètre donné, est une mesure qualitative ou quantitative de ce paramètre effectuée dans des conditions dites « contrôles » ou « normales », par exemple déterminée dans un échantillon de référence, ou déterminée dans un échantillon en l'absence d'un traitement présumé avoir un effet sur le paramètre.

Un échantillon convenant à l'invention peut être un échantillon de peau isolée prélevé chez un individu ou à partir d'un modèle de peau reconstruite *in vitro.*

Par exemple, une mesure de référence pour une séquence d'acides aminés ou une séquence d'acides nucléiques conforme à l'invention peut être une valeur quantitative ou qualitative relative à l'expression, la maturation ou l'activité desdites séquences déterminée dans un échantillon de peau physiologiquement saine, et présentant une desquamation normale ou régulière, ou déterminée dans un échantillon de peau, notamment présentant un trouble de la desquamation de la peau, avant un traitement cosmétique.

De préférence, une mesure de référence est une mesure statistique, c'est à dire ayant été répétée sur différents échantillons de sorte à obtenir une moyenne.

La mesure de référence peut être effectuée parallèlement ou séquentiellement à la mesure test.

Elle peut également être une mesure « historique », c'est-à-dire effectuée antérieurement à la mesure test, et stockée, par exemple dans une base de données, en vue d'une utilisation ultérieure.

Une comparaison de la mesure test à une mesure de référence, et une observation d'une déviation ou d'une absence de déviation entre les deux mesures, permet de tirer une information quant au paramètre mesuré, par exemple la diminution ou l'augmentation l'expression, de la maturation ou de l'activité d'une séquence d'acides aminés ou d'une séquence d'acides nucléiques conformes à l'invention.

Une telle information peut être ultérieurement mise en oeuvre pour déterminer la présence d'une desquamation normale ou régulière, ou *a contrario,* anormale ou irrégulière d'une peau.

Lorsqu'un biomarqueur de l'invention mis en oeuvre est une séquence d'acides aminés, une desquamation anormale ou irrégulière de la peau peut se traduire par une déviation de l'expression de cette séquence d'un facteur d'au moins 1,2, de préférence d'au moins 1,5, et de manière plus préférée d'au moins deux fois la valeur de référence normale.

L'invention peut également être effectuée sur un échantillon de peau, prélevé à partir d'un modèle cellulaire épidermique, ou d'une peau isolée reconstruite afin d'en qualifier l'état.

Selon encore un autre aspect, un biomarqueur de l'invention peut être utilisé pour sélectionner parmi un ensemble d'agents actifs pour prévenir et/ou traiter un trouble de la desquamation de la peau, un agent actif présumé exercer l'effet bénéfique maximal à l'égard dudit trouble.

L'ensemble des actifs dans lequel peut opérer une sélection d'une utilisation de l'invention peut être l'ensemble des actifs classiquement utilisé pour traiter un trouble de la desquamation. Plus particulièrement, un biomarqueur de l'invention peut être utilisé pour la sélection d'un agent actif le plus adapté au traitement d'un individu présentant un état pelliculaire du cuir chevelu. Dans un tel cas, les actifs qui seront considérés sont ceux habituellement mis en oeuvre pour le traitement des états pelliculaires.

Ainsi, un biomarqueur de l'invention peut avantageusement être utilisé pour mettre en place un service de conseil personnalisé auprès d'individu présentant un trouble de la desquamation de la peau, et en particulier présentant un état pelliculaire du cuir chevelu.

Selon le degré de déviation du biomarqueur par rapport à une référence normale, le conseiller pourra opérer un choix vers le produit le plus adapté de sorte à obtenir la meilleure correction du biomarqueur par rapport à la normale, tout en réduisant le risque de survenue d'effets secondaires ou indésirables qui pourraient s'avérer être délétères à la bonne compliance du traitement.

Selon un autre aspect, un biomarqueur de l'invention peut également être mis en oeuvre dans un processus de sélection et de constitution de groupe d'individu pour la réalisation d'essais cliniques dédiés à l'évaluation de l'efficacité d'un produit présumé être actif à l'égard d'une desquamation anormale de la peau, et de préférence d'un état pelliculaire du cuir chevelu.

Avantageusement, pour obtenir des résultats significatifs à l'issue d'un essai clinique, un biomarqueur de l'invention peut être mis en oeuvre afin de définir des groupes d'individus homogènes présentant le même degré de déviation du biomarqueur par rapport à une valeur de référence.

Le présent texte décrit un procédé, en particulier *in vitro* ou *ex vivo,* pour caractériser un état de desquamation de la peau.

Un tel procédé permet avantageusement de caractériser un trouble de la desquamation de la peau résultant d'une desquamation anormale ou irrégulière, et plus particulièrement un état pelliculaire du cuir chevelu.

Selon un autre mode de réalisation, le présent texte décrit un procédé cosmétique, ou non thérapeutique, en particulier *in vitro* ou *ex vivo,* pour caractériser l'efficacité d'un traitement cosmétique de la peau, et de préférence d'un traitement d'une desquamation anormale de la peau, et de préférence un état pelliculaire du cuir chevelu chez un individu en ayant besoin, comprenant au moins les étapes consistant à :
a) effectuer, avant la mise en oeuvre du traitement cosmétique, dans un premier échantillon de peau isolé, et de préférence de cuir chevelu isolé, prélevé chez ledit individu, au moins une première mesure qualitative ou quantitative de l'expression, de la maturation ou de l'activité d'au moins une séquence d'acides aminés de l'invention ou d'au moins une séquence d'acides nucléiques de l'invention,
b) effectuer, après la mise en oeuvre du traitement cosmétique, dans un second échantillon de peau isolé, et de préférence de cuir chevelu isolé, prélevé chez ledit individu, au moins une seconde mesure, qualitative ou quantitative de l'expression, de la maturation ou de l'activité de ladite séquence d'acides aminés de l'invention ou de ladite séquence d'acides nucléiques de l'invention, et
c) comparer les première et deuxième mesures, notamment afin d'en déduire une information relative à un effet au moins de la mise en oeuvre du traitement cosmétique.

Il va de soit que les mesures effectuées aux étapes a) et b) doivent être comparables l'une à l'autre, et donc être relatives au même paramètre.

De manière préférée, un tel procédé permet de mettre en évidence un effet d'un traitement cosmétique susceptible de normaliser une desquamation anormale ou irrégulière de la peau, et en particulier un état pelliculaire du cuir chevelu.

De manière préférée encore, un tel procédé permet de caractériser l'efficacité d'un traitement cosmétique, et en particulier un traitement d'un état pelliculaire du cuir chevelu.

Une mesure qualitative ou quantitative d'une expression, de la maturation ou d'une activité d'une séquence d'acides nucléiques de l'invention peut être déterminée par toute méthode connue de l'homme de l'art.

A titre d'exemple de méthodes convenant à l'invention, il peut être fait mention de la réaction de polymérisation en chaîne (PCR), quantitative (Q-PCR) ou non, en présence ou non de transcriptase inverse (RT-PCR ou Q-RT-PCR), du Northern-blot, de la méthode « ribonuclease protection assay », des méthodes avec des puces à ADN, des méthodes avec des puces transcriptomiques, des méthodes avec des puces à oligonucléotides, des méthodes d'hybridation *in situ.*

A titre d'exemple d'agents convenant à la détection d'une séquence d'acides nucléiques de l'invention, et en particulier d'une séquence d'ARNm, il peut être fait mention de sondes d'acides nucléiques marquées pouvant s'hybrider à une séquence d'acides nucléiques de l'invention.

Une telle sonde d'acide nucléique peut être aisément obtenue par toute méthode connue de l'homme de l'art.

Ainsi, les séquences d'acides nucléiques telles que décrites dans le présent texte peuvent être utilisées pour réaliser des amorces oligonucléotidiques sens et/ou anti-sens, qui s'hybrident dans des conditions de forte stringence à au moins une des séquences SEQ ID NO: 1, SEQ ID NO: 2, SEQ ID NO: 3, SEQ ID NO: 4, SEQ ID NO: 5, SEQ ID NO: 6, ou SEQ ID NO: 7, un analogue ou un fragment celle-ci.

L'expression d'une séquence d'acide nucléique peut également être déterminée, de manière indirecte, par la détermination de l'expression de la séquence d'acides aminées codée par ladite séquence, au moyen de toute technique connue dans le domaine, telle que le Western-Blot, l'ELISA, la méthode de BRADFORD ou de LOWRY, ou comme indiqué ci-après l'électrophorèse 2D.

Une mesure qualitative ou quantitative, d'une expression, de la maturation, ou d'une activité d'une séquence d'acides aminés de l'invention peut être effectuée au moyen de toute méthode connue de l'homme de l'art.

A titre de méthodes de détection de l'expression, de la maturation, ou de l'activité d'une séquence d'acides aminés, il peut être fait mention du Western-blot, du Slot-blot, du Dot-blot, des méthodes ELISA (Enzyme Linked Immuno-Sorbent Assay) de type singleplex ou multiplex, des méthodes de protéomique ou de glycomique, protéomique différentiel par approche 2D-électrophorèse, de coloration de polypeptides dans un gel de polyacrylamide par un colorant à base d'Argent, par le bleu de Coomassie ou par le SYPRO, de l'immunofluorescence, de l'absorption UV, de méthodes immunohistochimiques en microscopie classique, électronique ou confocale, de FRET (fluorescence resonance energy transfer/transfert d'énergie par résonance de fluorescence), des méthodes de TR-FRET (time resolved FRET/FRET en résolution de temps), des méthodes de FLIM (fluorescence lifetime imaging microscopy/imagerie par microscopie en temps de fluorescence), des méthodes de FSPIM (fluorescence spectral imaging microscopy/imagerie par microscopie en spectre de fluorescence), des méthodes de FRAP (fluorescence recovery after photobleaching/recouvrement de fluorescence après photoblanchiment), des méthodes par gène rapporteur, des méthodes d'AFM (atomic force microscopy/microscopie par force atomique), des méthodes de résonance plasmonique de surface, des méthodes de microcalorimétrie, des méthodes de cytométrie en flux, des méthodes de biosenseurs, des méthodes de radioimmuno-essais (RIA), des méthodes de focalisation isoélectrique, et des tests enzymatiques, des méthodes mettant en oeuvre des puces à peptides, des puces à sucre, des puces d'anticorps, des méthodes de spectrométrie de masse, des méthodes de spectrométrie de type SELDI-TOF (Ciphergen), ou des méthodes quantification par spectrométrie de masse de type MRM (Multiple Reaction Monitoring).

De façon plus générale, des méthodes de dosage immunoenzymatiques à partir de solutions de protéines, plus quantitatives et sensibles peuvent en particulier être utilisées. Ces méthodes de type ELISA associent des couples d'anticorps de capture et de détection spécifique de l'antigène ciblé. Des anticorps commerciaux ou des anticorps polyclonaux, monoclonaux ou recombinants développés spécifiquement peuvent être utilisés. Des techniques ELISA multiplexes de grande capacité peuvent également être mises en oeuvre. On peut ainsi citer l'approche multiplexe de type anticorps sur billes Luminex (par exemple Bioplex de Bio-Rad), l'approche singleplex ou multiplex par chemiluminescence de la société Mesoscale Discovery (MSD), ou de type anticorps sur surface plane (« antibodies-arrays ») (par exemple approche proposée par la société MesoScale Discovery).

En particulier, il peut être avantageux de détecter l'expression d'une séquence d'acides aminés de l'invention au moyen d'un anticorps, le cas échéant sous une forme marquée. Un tel anticorps peut être marqué au moyen d'une substance détectable directement ou détectable par réaction avec un autre réactif.

Par « anticorps », on entend désigner de manière générale des anticorps monoclonaux ou polyclonaux, ainsi que des fragments d'immunoglobuline susceptibles de lier un antigène et qui peuvent être produits par toute technique de génie génétique connue de l'homme de l'art ou par coupure enzymatique ou chimique d'anticorps intact.

Un anticorps susceptible d'être utilisé à titre d'outil d'évaluation d'un état d'un épiderme peut être obtenu par tout procédé connu de l'homme de l'art, tel que décrit dans « Antibodies: A Laboratory Manual », Cold Spring Harbor Laboratory Press, Cold Spring Harbor, N.Y. (1990).

Selon un mode de réalisation préféré, il peut être avantageux de détecter l'expression d'une séquence d'acides aminés de l'invention au moyen d'une méthode protéomique différentielle par approche électrophorèse 2D ou par une approche de marquage isobarique iTRAQ, par une approche label free Spectral Counting ou de type SILAC si il s'agit de cultures cellulaires.

Une telle méthode est connue de l'homme de l'art et peut avantageusement être mise en oeuvre comme décrit dans les exemples ci-après.

De manière particulière, par « activité » à l'égard d'une séquence d'acides aminés de l'invention on entend une activité protéolytique comme indiquée précédemment, ou une activité de réduction, de prévention ou de traitement d'un trouble de la desquamation d'une peau, par exemple tel que défini précédemment, et notamment un état pelliculaire du cuir chevelu.

Une telle activité peut être déterminée par toute méthode connue de l'homme de l'art, comme par exemple, par évaluation d'une activité protéolytique sur un substrat habituel de l'IDE tels que l'insuline, le glucagon ou la protéine β-amyloïde.

De manière préférée, la détermination d'un état de la peau ou la caractérisation de l'efficacité d'un traitement cosmétique de la peau peuvent être effectuées par mesure de la variation de l'expression d'une séquence d'acides aminées de l'invention, et de préférence représentée par une séquence choisie parmi SEQ ID NO: 8 à SEQ ID NO: 14, un analogue ou un fragment de celle-ci.

Les procédés de l'invention sont particulièrement avantageux dans la mesure où leur mise en oeuvre ne nécessite pas de recourir à une technique invasive. Un échantillon d'épiderme peut ainsi être obtenu par des techniques dites de « stripping » et directement analysé par une technique d'analyse conventionnelle connue de l'homme de l'art.

Ces strippings sont des surfaces collantes appliquées à la surface de l'épiderme comme le Blenderm® de 3M, le D'squam (adhésif commercial de CuDERM), la colle cyanoacrylate, la méthode du « stripping » vernis ou les cornéodisques. Grâce à ces « strippings », les cornéocytes adhérents et le contenu de leurs espaces intercellulaires peuvent être prélevés et soumis par la suite à une extraction permettant d'avoir accès au contenu protéique.

Le prélèvement d'un échantillon convenant à un procédé de l'invention peut aussi être effectué de façon plus directe par « lavages » de la surface cutanée, au moyen par exemple d'accessoires de type turbine à ailette, de type cellule à spirale telle que décrit dans le brevet FR 2 667 778 associée à un circuit fluidique, ou simplement par ajout/prélèvement d'une goutte de tampon à la surface de la peau.

A titre indicatif, d'autres méthodes de prélèvement adaptées à la mise en oeuvre de l'invention peuvent être mentionnées, telles que des méthodes par grattage de la partie supérieure du *stratum corneum* au moyen d'un système bilames. Cette technique permet de recueillir des squames qui peuvent ensuite être directement analysés par différentes techniques pour déterminer les taux en minéraux, aminoacides ou lipides.

Avantageusement, un des marqueurs de l'invention peut être mise en oeuvre à des fins de sélection préclinique, plus efficace et plus rigoureuse, d'individus, en vue d'évaluer l'efficacité de traitement ou d'actif cosmétique pour le soin de la peau, et notamment du cuir chevelu.

Egalement, un biomarqueur de l'invention peut avantageusement être mis en oeuvre comme indiqué précédemment pour évaluer l'efficacité d'un agent actif, *in vitro, ex vivo* ou *in vivo.*

Egalement, un biomarqueur de l'invention peut être mis en oeuvre pour établir un conseil personnalisé d'un traitement cosmétique pour un individu en fonction de son profil d'expression de biomarqueurs cutanés.

### Criblage

Selon un de ses aspects, le présent texte décrit l'utilisation d'un biomarqueur de l'invention, pour cribler ou dans un procédé de criblage, en particulier *in vitro* ou *ex vivo,* d'agents actifs ou de traitements physiques pour le soin de la peau.

Les agents actifs ou les traitements physiques criblés peuvent notamment être utilisés pour prévenir et/ou traiter une desquamation anormale ou irrégulière de la peau, et de préférence pour prévenir et/ou traiter un état pelliculaire du cuir chevelu.

Une utilisation ou un procédé de criblage peuvent comprendre la comparaison d'une mesure de l'activité, de l'expression ou de la maturation d'une séquence d'acide aminés ou d'une séquence d'acides nucléiques conforme à l'invention à une mesure de référence.

Une mesure de référence peut être telle que définie précédemment.

En particulier une mesure de référence peut être une valeur quantitative ou qualitative relative à l'expression, la maturation ou l'activité desdites séquences déterminée dans un échantillon en l'absence d'agent actif ou de traitement physique testé.

Ainsi, une mesure de référence peut être obtenue en réitérant les étapes d'un procédé de l'invention, et notamment les étapes a), b) et c) d'un procédé de l'invention tel que défini précédemment, en l'absence d'agents actifs, ou de traitements physiques à tester.

Une comparaison de la mesure test à une mesure de référence, et une observation d'une déviation ou d'une absence de déviation entre les deux mesures, permet de tirer une information quant à l'effet de l'agent actif ou du traitement physique testé.

La détermination quantitative ou qualitative de l'expression, de la maturation ou de l'activité d'une séquence d'acides aminés ou d'une séquence d'acides nucléiques de l'invention peut être effectuée par toute méthode connue de l'homme de l'art, et notamment comme décrit précédemment.

Selon un mode de réalisation, le criblage d'un agent actif ou d'un traitement physique susceptible de moduler l'activité d'une séquence d'acides aminées de l'invention peut se faire par mesure de l'activité ou de l'expression d'une molécule cible appartenant aux voies de signalisation ou de métabolisme dans lesquelles peut être impliqué ladite d'une séquence d'acides aminées, tel que par exemple un système de gène reporteur.

Selon un mode de réalisation, un procédé de l'invention peut être mis en oeuvre dans un système acellulaire, i.e. dans un système ne comprenant pas de cellules mais reproduisant des fonctions cellulaires, ou dans un échantillon cellulaire isolé.

Un procédé conforme à l'invention peut être effectué sur un échantillon cellulaire isolé, un échantillon acellulaire, sur une séquence d'acides aminés isolée ou sur une séquence d'acides nucléiques isolée de l'invention. Ces échantillons ou séquences peuvent être obtenus par biopsie cutanée, à partir de cellules en culture, notamment à partir d'un modèle épidermique, ou d'un prélèvement de surface cutané non-invasif, notamment par adhésif (« tape-stripping ») de *stratum corneum* ou par simple lavage, comme décrit précédemment, voire, pour ce qui est des séquences, par synthèse.

La mise en oeuvre de ces échantillons ou séquences pour la réalisation d'un criblage d'agent actifs ou de traitement physique conformément à l'invention relève des connaissances générales de l'homme de l'art dans le domaine.

Avantageusement, à titre d'échantillon cellulaire convenant à l'invention, on peut mentionner un échantillon de kératinocytes ou tout autre type cellulaire de la peau exprimant une séquence d'acides aminés de l'invention.

De manière préférée, le criblage d'un agent actif ou d'un traitement physique peut être effectuée par mesure de la variation de l'expression ou de l'activité, en présence et en absence de l'agent actif ou du traitement physique criblé, d'une séquence d'acides aminées de l'invention, et de préférence représentée par une séquence choisie parmi SEQ ID NO: 8 à SEQ ID NO: 14, un analogue ou un fragment de celle-ci, et de préférence choisi parmi SEQ ID NO: 9 à SEQ ID NO: 14, un analogue ou un fragment de celle-ci.

### Agent modulateur

Au sens de la présente invention, on entend par « agent modulateur » ou « agent actif ou un traitement physique susceptible de moduler l'expression, la maturation ou l'activité d'une séquence d'acides aminées ou d'une séquence d'acides nucléiques conformes à l'invention », tout composé ou phénomène physique susceptible d'agir, directement ou indirectement, sur au moins une séquence d'acides aminées ou une séquence d'acides nucléiques conformes à l'invention, ou sur un élément d'une voie de signalisation intra ou extra-cellulaire, ou d'une voie métabolique, ou de régulation de la transcription et/ou de la traduction impliquant ladite séquence d'acides aminées ou ladite séquence d'acides nucléiques.

Les agents modulateurs ou les traitements physiques préférés selon le présent texte peuvent être des composés ou traitements physiques agissant directement sur au moins une séquence d'acide aminés de l'invention ou au moins une séquence d'acide nucléique de l'invention en vu de moduler leur expression, ou leur maturation, ou leur activité.

Au sens de l'invention, on entend par « moduler », au regard d'un effet donné, l'action de stimuler ou d'inhiber cet effet.

Les agents actifs ou les traitements physiques issus d'un criblage décrit dans le présent texte peuvent être avantageusement utilisés à des fins cosmétiques, notamment au regard des troubles de la desquamation de la peau, notamment des troubles des propriétés barrières de la peau, et en particulier des troubles des propriétés barrières du cuir chevelu.

Selon un mode de réalisation préféré, un agent modulateur de l'invention est un agent inhibiteur de l'activité, de l'expression ou de la maturation d'une séquence d'acides aminés de l'invention. De manière plus préférée encore, un agent modulateur est un agent inhibiteur de l'activité enzymatique d'une séquence d'acides aminés de l'invention.

Un agent modulateur inhibiteur de l'activité enzymatique d'une séquence d'acides aminés de l'invention peut être choisi parmi l'ATP, l'ADP, le N-éthylmaléimide, la 1,10-phénantroline, la bacitracine, l'insuline, l'hGH, le PMSF, des acides gras tels que l'acide palmitique, l'acide linoléique, le palmitoyl-CoA, le linoléoyl-CoA, des ions métalliques tels que le Mg²⁺, le Mn²⁺, le Zn²⁺, le Li⁺, la dynorphine, l'ubiquitine, des agents chélateurs de métaux, notamment des chélateurs du Zn tels que l'EDTA, des peptides hydroxamates de formule générale suivante : 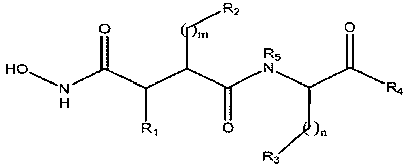 dans laquelle R₁, R₂, R₃, R₄, R₅ et R₆ sont indépendant l'un de l'autre, et
dans laquelle
- R₁ est H, OH, O-alkyle(C₁-C₆) ou alkyle(C₁-C₆),
- R₂ est un aryle(C₅-C₁₀), un hétéroaryle(C₅-C₁₀) comprenant au moins un N, O ou S, un phényle, un napht-1-yl, un napht-2-yl, le cas échéant substitué par un halogène, notamment Cl ou F, un alkyle(C₁-C₆), un O-alkyle(C₁-C₆), un nitrile, un CN, un aryle(C₅-C₁₀), un hétéroaryle(C₅-C₁₀) comprenant au moins un N, O ou S, ou un CO₂H
- R₃ est NHC(=NH)NH₂, NH₂, NHC(O)alkyle(C₁-C₆), ou NHC(O)aryl(C₅-C₁₀),
- R₄ est [C(=O)CH(CH₂)ₒR₇NH]ₚH ou [C(=O)CH(CH₂)ₒR₇NH]pC(=O)Me avec R₇ étant 4-hydroxyphényl, CO₂H, indol-3-yl, ou un phényle, o variant de 0 à 3, et p variant de 0 à 2,
- R₅ est H ou Me,
- R₆ est H ou Me,
- m varie de 0 à 3 et n varie de 0 à 3.

Les peptides hydroxamates convenant à l'invention sont plus particulièrement décrits dans WO 2008/156701.

De manière préféré, un peptide hydroxamate peut être un composé de formule générale (I) précédente, dans laquelle R₂ est un napht-2-yl, et R₃ est NHC(=NH)NH₂, et R₁, R₄, R₅ et R₆ sont tels que définis précédemment

De manière préférée encore, R₂ peut être un aryle, hétéroaryle, un aryle substitué, un hétéroaryle substitué, un aryle halo-substitué tel que un 2-fluorophényl, un 3-fluorophényl, un 4-fluorophényl, un hétéroaryle halo-substitué, un aryle alkyl ou aryl-substitué, un hétéroaryle alkyl ou aryl-substitué, tel qu'un 2-tert-butyl, 3-tert-butyl, 4-tert-butyl, phényl, un 1-naphtyl, 2-naphtyl, 2-benzothiophène, -trans-CH=CH-phenyl, 2-phenyl, 3-phenyl, ou 4-phenyl.

De manière plus particulière encore, un peptide hydroxamate peut être choisi parmi : 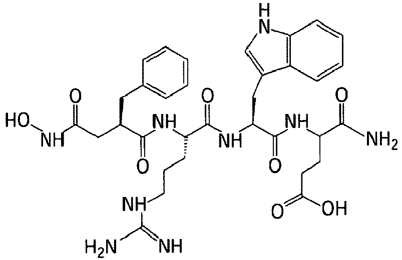 ou 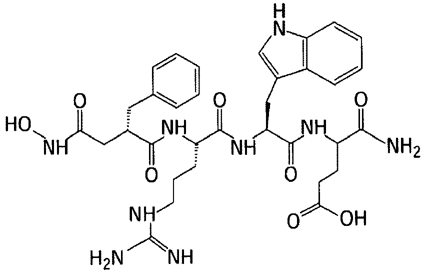 ou 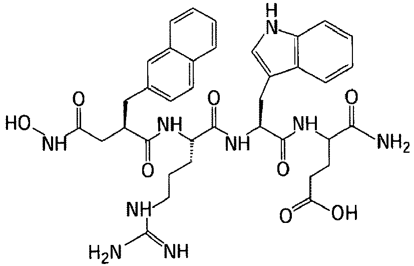 ou

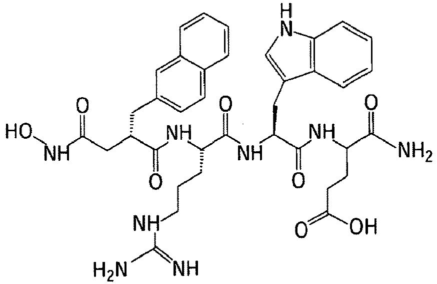

Outre les agents inhibiteurs précédemment cités, on peut également citer, à titre d'inhibiteurs de l'IDE, des composés de type nucléosides phosphates ou des fragments peptidiques de l'IDE, ainsi que des ARN interférents, des aptamères à ADN ou ARN ou des anticorps bloquant le site actif ou l'exosite de l'enzyme.

Selon un mode de réalisation, un agent modulateur, notamment un agent inhibiteur de l'activité, de l'expression ou de la maturation d'une séquence d'acides aminés de l'invention peut avantageusement être mis en oeuvre pour prévenir et/ou traiter un état pelliculaire du cuir chevelu. De manière préférée encore, le présent texte décrit la mise en oeuvre d'un agent modulateur inhibiteur de l'activité enzymatique d'une séquence d'acides aminés de l'invention pour prévenir et/ou traiter un état pelliculaire du cuir chevelu.

Selon un autre aspect, le présent texte décrit également des agents modulateurs de l'invention choisis parmi des agents activateurs de l'activité, de l'expression ou de la maturation d'une séquence d'acides aminés de l'invention et notamment activateurs de l'activité enzymatique d'une séquence d'acides aminés de l'invention.

Selon un mode de réalisation, un agent modulateur activateur de l'activité enzymatique peut être choisi parmi la suramine, la somatostatine, la bradykinine, la β-endorphine, la dynorphine, l'ATP, l'iPPP, l'ADP, l'AMP, des acides gras, notamment tels que l'acide docosahexaénoïque, et les composés de formules suivantes : 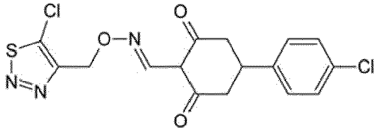 ou

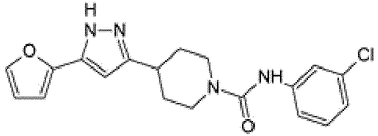

Ces composés sont notamment décrits dans Cabrol et al. (PLoSOne, 2009).

Parmi les agents activateurs de l'activité enzymatique de l'IDE, on peut également citer des nucléosides phosphates, des fragments peptidiques de l'IDE, ou des anticorps activateurs de l'enzyme.

A titre d'agent modulateur susceptible d'être criblé selon une utilisation ou un procédé décrit dans le présent texte, on peut également mentionner les anticorps, notamment issus de banques d'anticorps recombinants, par exemple de la société ANTIBODIES BY DESIGN, et aptes à bloquer le site actif ou l'exosite de l'enzyme, ou à activer l'enzyme, par exemple par effet allostérique, ou les ARN interférents, tels que les siARN, les miARN ou les shARN, ou les aptamères à ARN ou à ADN.

### Compositions

Le présent texte décrit également des compositions, notamment cosmétiques, comprenant dans un milieu physiologiquement, ou cosmétiquement, acceptable une quantité efficace d'au moins une séquence d'acides aminés de l'invention, ou d'au moins une séquence d'acides nucléiques de l'invention, ou d'au moins un agent actif apte à moduler l'activité, l'expression ou la maturation de ladite séquence d'acides aminés ou de ladite séquence d'acides nucléiques.

Plus particulièrement, le texte décrit une composition cosmétique comprenant un peptide représenté par une séquence d'acides aminés choisie parmi SEQ ID NO: 9 à SEQ ID NO: 14, un analogue ou un fragment de celle-ci ou une séquence d'acides nucléiques codant pour un tel peptide.

Au sens de la présente invention, on entend désigner par « milieu physiologiquement acceptable », un milieu convenant à l'administration d'une composition par voie topique sur la peau, le cuir chevelu, ou les lèvres, ou par voie orale ou par voie parentérale, telle que la voie intradermique ou sous-cutanée.

Une composition de l'invention peut contenir des adjuvants habituels dans le domaine considéré, tels que des gélifiants hydrophiles ou lipophiles, des additifs hydrophiles ou lipophiles, des conservateurs, des antioxydants, des solvants, des parfums, des charges, des filtres, des absorbeurs d'odeurs et des matières colorantes.

Les quantités des différents constituants des compositions selon l'invention sont celles classiquement utilisées dans les domaines considérés.

La quantité de séquence d'acides aminés, ou de séquence d'acides nucléiques de l'invention, ou d'agent actif contenue dans une composition de l'invention, encore dite « quantité efficace » est, bien entendu, fonction de la nature de l'actif et de l'effet recherché et peut donc varier dans une large mesure.

Pour donner un ordre de grandeur, une composition peut contenir une séquence d'acides aminés, ou une séquence d'acides nucléiques, ou un agent actif conformes à l'invention en une quantité représentant de 0,00001 % à 50 % du poids total de la composition, en particulier en une quantité représentant de 0,001 % à 10 % du poids total de la composition, et plus particulièrement en une quantité représentant de 0,1 % à 1 % du poids total de la composition.

Selon un autre mode de réalisation, une composition cosmétique de l'invention peut comprendre, en outre, au moins un agent actif cosmétique et/ou thérapeutique additionnel.

### Actifs additionnels

Comme exemples d'agents actifs additionnels utilisables dans le cadre de la présente invention, il peut être fait mention d'huiles cosmétiques, telles que les huiles de silicone, les huiles végétales de type triglycérides, les huiles hydrocarbonées telles que l'huile de PARLEAM et les esters d'acides gras et d'alcool gras.

Il peut également être possible d'utiliser d'autres agents actifs permettant d'améliorer l'état de la peau et/ou de ses annexes, tels que des actifs hydratants ou humidifiants ou des agents actifs permettant d'améliorer la barrière lipidique naturelle, tels que des céramides, des sulfates de cholestérol et/ou des acides gras, et leurs mélanges.

Il peut également être possible d'utiliser des enzymes ayant une activité sur la peau et/ou de ses annexes, telles que des protéases, des lipases, des glucosidases, des amidases, des cérébrosidases et/ou des mélanases, et leurs mélanges.

Comme autres exemples d'agents actifs convenant à la mise en oeuvre de la présente invention figurent : des actifs analgésiques, des actifs anti-levures, des actifs antibactériens, des actifs antiparasitaires, des actifs antifongiques, des actifs antiviraux, des actifs anti-inflammatoires stéroïdiens, des actifs anesthésiques, des actifs antiprurigineux, des actifs kératolytiques, des actifs anti-radicaux libres, des actifs anti-séborrhéiques, des actifs antipelliculaires, des actifs anti-acnéiques, des actifs visant à prévenir le vieillissement de la peau et/ou à améliorer son état, des actifs anti-dermatites, des actifs anti-irritants, des actifs immuno-modulateurs, des actifs pour le traitement de la peau sèche, des actifs anti-transpirants, des actifs anti-psoriatiques, des actifs protecteurs contre les UV, des actifs antihistaminiques, des actifs cicatrisants, des actifs auto-bronzants, des antioxydants tels que le thé vert ou des fractions actives de celui-ci, la glycérine, la laponite, la caféine, des huiles essentielles aromatiques, des colorants, des actifs dépigmentants, des lipo-régulateurs, des actifs adoucissants, rafraîchissants, déodorants, insensibilisants, blanchissants, nourrissants, des actifs diminuant la différenciation et/ou la prolifération et/ou la pigmentation cutanée, et leurs mélanges.

Egalement, à titre d'actifs additionnels, on peut citer le Tween 20 ou d'autres détergents doux, des agents chélateurs, des probiotiques, du Zn-pyrithione ou d'autres antifongiques, l'acide élagique, des agents prodesquamants ou/et de peeling, des composés anti-prurit, des polyphénols et leurs dérivés, des sucres et des sucres réducteurs, des anti-inflammatoires, des anti-sudoraux, ou des anti-sébacés, etc.

A titre d'actifs additionnels susceptibles de convenir plus particulièrement à l'invention, on peut mentionner également des microorganismes probiotiques, des actifs prébiotiques, des actifs favorisant la synthèse de facteur de défense de la peau, des actifs permettant de rétablir l'équilibre différentiation/prolifération des cellules de l'épiderme, en particulier tels que des actifs de type retinol ou retinol-like, ou des actifs hydratants.

### Utilisation cosmétique

Le présent texte décrit l'utilisation cosmétique d'une quantité efficace d'au moins un agent modulateur de l'activité, de l'expression ou de la maturation de ladite séquence d'acides aminés ou de ladite séquence d'acides nucléiques, et notamment un agent modulateur tel que défini précédemment, à titre d'agent actif pour prévenir et/ou traiter une desquamation anormale ou irrégulière de la peau, en particulier un trouble des propriétés barrières du cuir chevelu, et de préférence un état pelliculaire du cuir chevelu.

Selon un mode de réalisation préféré, une telle utilisation peut mettre en oeuvre un agent modulateur inhibiteur de l'activité, de l'expression ou de la maturation d'une séquence d'acides aminés de l'invention, et de préférence inhibiteur de l'activité enzymatique d'une séquence d'acides aminés de l'invention. Un tel agent peut notamment être choisi parmi les agents inhibiteurs définis précédemment.

Selon un autre mode de réalisation, une telle utilisation peut mettre en oeuvre un agent modulateur activateur de l'activité enzymatique d'une séquence d'acides aminés de l'invention avantageusement choisi parmi les agents activateurs définis précédemment.

Selon un autre aspect, le présent texte décrit un procédé cosmétique, ou non thérapeutique, pour prévenir et/ou traiter une desquamation anormale ou irrégulière de la peau, et en particulier un état pelliculaire du cuir chevelu, chez un individu en ayant besoin.

Un tel procédé peut comprendre au moins une étape consistant à administrer audit individu, au moins une composition comprenant à titre d'agent actif au moins un agent modulateur de l'activité, de l'expression ou de la maturation desdites séquences de l'invention, notamment tels que définis ci-avant.

De préférence, un agent modulateur considéré est un agent inhibiteur de l'activité enzymatique d'une séquence d'acides aminés de l'invention, notamment tel que défini précédemment.

Un tel procédé ou une telle utilisation permet de prévenir et/ou traiter un trouble de la desquamation de la peau, notamment tels que définis précédemment et de manière préférée, un état pelliculaire du cuir chevelu.

Un procédé de l'utilisation de l'invention peut permettre de réduire le nombre et la taille des squames ou pellicules.

Avantageusement, un cuir chevelu peut voir son aspect esthétique amélioré et ses propriétés de fonction barrières restaurées suite à la mise en oeuvre d'une utilisation ou d'un procédé décrit précédemment.

De manière générale, un tel procédé ou une telle utilisation permettent de renforcer les propriétés barrières de la peau, et en particulier du cuir chevelu.

De manière préférée, un tel procédé peut comprendre l'application par voie topique sur au moins une partie de la peau d'un individu en ayant besoin, en particulier sur le cuir chevelu, d'au moins une couche d'une composition topique de l'invention.

Un procédé cosmétique par voie topique tel que décrit peut avantageusement comprendre l'application d'une composition de l'invention, en association de manière simultanée, successive ou séparée dans le temps avec une composition cosmétique ou dermatologique additionnelle distincte de la composition de l'invention et destinée au soin et/ou au maquillage de la peau, et de préférence au soin du cuir chevelu.

Selon un autre mode de réalisation préféré, un tel procédé cosmétique peut être mis en oeuvre par voie orale, notamment par administration d'au moins une composition alimentaire ou diététique à visée cosmétique.

Selon un autre mode de réalisation préféré, un tel procédé cosmétique peut être mis en oeuvre par voie parentérale. La mise en oeuvre par voie parentérale d'un tel procédé cosmétique est effectuée à l'exclusion de toute intervention chirurgicale et ne vise qu'à exercer un traitement de surface de la peau à des fins esthétiques.

Ainsi, un tel procédé cosmétique par voie parentérale est mis en oeuvre par toute technique d'injection ou dispositif convenant à une injection intra-épidermique et/ou intradermique et/ou sous-cutanée.

Une telle administration peut être effectuée, par exemple, par mésothérapie.

Un procédé cosmétique par voie parentérale ne se traduit donc que par une effraction superficielle de la peau et sort donc de tout cadre médical ou thérapeutique.

Pour la voie parentérale, on pourra alternativement privilégier l'administration par patch à visée systémique.

Un procédé cosmétique tel que décrit peut être mis en oeuvre de façon journalière, par exemple à raison d'une administration unique par jour, ou d'une administration fractionnée en deux ou trois fois par jour, par exemple une fois le matin et une fois le soir.

Un procédé cosmétique selon l'invention peut être mis en oeuvre sur une période de temps variant d'une semaine à plusieurs semaines, voire plusieurs mois, cette période pouvant par ailleurs être répétée après des périodes de non traitement pendant plusieurs mois, voire plusieurs années.

A titre d'exemple, de procédé cosmétique décrit ci-dessus on peut prévoir une administration d'une composition de l'invention, par exemple, à raison de 1, 2 ou 3 fois par jour, ou plus, et généralement sur une durée prolongé d'au moins 4 semaines, voire 4 à 15 semaines, avec le cas échéant une ou plusieurs périodes d'interruption.

### Peau reconstruite

Selon un autre aspect, le présent texte décrit l'utilisation d'une quantité efficace d'au moins une séquence d'acides aminés de l'invention, ou d'au moins une séquence d'acides nucléiques de l'invention, ou d'au moins un agent modulateur pour préparer une peau reconstruite isolée.

L'intérêt de développer des modèles organotypiques les plus proches de conditions *in vivo* est grand pour l'évaluation sécurité et efficacité des actifs et formules à applications cosmétiques et dermatologiques.

L'utilisation d'au moins une séquence d'acides aminés de l'invention, ou d'au moins une séquence d'acides nucléiques de l'invention, ou d'au moins un agent modulateur dans un milieu de culture est susceptible d'améliorer la qualité des modèles développés.

Egalement, une utilisation de l'invention peut permettre l'obtention de modèle de peau reconstruite isolée reproduisant de la desquamation de la peau. En particulier, la mise en oeuvre d'un agent modulateur activateur, tel que défini précédemment, peut être considéré pour la préparation d'un modèle de peau reproduisant une desquamation, de préférence reproduisant en état pelliculaire.

Selon un autre aspect, le présent texte décrit un procédé de préparation d'un modèle cellulaire épithélial pluristratifié isolé, et de préférence une peau reconstruite isolée comprenant, au moins l'étape de mettre en contact au moins une quantité efficace d'au moins une séquence d'acides aminés, ou d'au moins une séquence d'acides nucléiques conformes à l'invention, ou d'au moins un agent modulateur avec des cellules susceptibles de générer une peau reconstruite isolée, et notamment des kératinocytes.

Un modèle de peau reconstruite peut comprendre différents types cellulaires, tels des kératinocytes, des fibroblastes, des cellules de Langerhans et des mélanocytes. Les cellules de type fibroblastes peuvent être irradiées ou non.

Avantageusement, un modèle de peau reconstruite peut être mis en oeuvre à titre de modèle d'un cuir chevelu. Un modèle de cuir chevelu reconstruit isolé peut avantageusement être mis en oeuvre à des fins de criblage de nouveaux actifs convenant au soin du cuir chevelu, et plus particulièrement de nouveaux actifs antipelliculaires.

De tels modèles et leur préparation sont connus de l'homme de l'art.

Selon encore un autre aspect, le présent texte décrit également un procédé de préparation d'un modèle cellulaire épithélial pluristratifié, de préférence un modèle de peau reconstruite, comprenant au moins une étape de mise en culture de cellules d'au moins un type cellulaire dudit modèle, lesdites cellules ayant été génétiquement modifiées pour supprimer ou pour augmenter l'expression d'une séquence d'acides aminés ou d'une séquence d'acide nucléiques de l'invention.

L'obtention de cellules génétiquement modifiées pour supprimer l'expression d'une séquence d'acides aminés ou d'une séquence d'acide nucléiques de l'invention, cellules dites « knock-out », peut être réalisée par toute méthode connue de l'homme de l'art.

A titre d'exemple, de telles cellules peuvent être obtenues par transfection et recombinaison homologue d'un fragment d'acide nucléique venant s'insérer dans ou prendre la place du gène exprimant la séquence d'acides aminés dont l'expression est à supprimer.

Egalement, il peut être possible de supprimer l'expression d'un gène donné par transfection dans la cellule d'une séquence d'acide nucléique codant pour un ARN interférant spécifique de l'ARNm issu du gène dont l'expression est à supprimer.

L'obtention de cellules génétiquement modifiées pour augmenter l'expression d'une séquence d'acides aminés ou une séquence d'acides nucléiques de l'invention peut être réalisée par toutes méthodes connues de l'homme de l'art.

A titre d'exemples, de telles cellules peuvent être obtenues par transfection de plasmide comprenant un gène codant pour une séquence d'acides aminés de l'invention sous le contrôle d'un promoteur. Le promoteur utilisé peut permettre l'expression du gène de manière inductible ou constitutive, ou de manière tissu-spécifique.

Egalement, il est possible d'utiliser différents vecteurs de transfection permettant l'introduction du gène à faire exprimer dans les chromosomes des cellules à transfecter.

Toutes ces techniques de biologie moléculaire permettant de réprimer ou d'augmenter l'expression d'un gène sont connues de l'homme de l'art et ne nécessitent pas de développement spécifique.

### FIGURES

**Figure 1** **:** représente l'agrandissement d'un spot protéique obtenu par analyse protéomique différentielle par approche électrophorèse 2D identifié comme étant l'IDE.
**Figure 2** **:** illustre la représentation graphique de l'intensité du volume des spots observés en Figure 1.
**Figure 3** **:** représente l'immunodétection par Western-Blot de l'IDE.
**Figure 4** **:** représente l'intensité moyenne des bandes détectées lors du Western-Blot illustré par la figure 3.

Au sens de la présente invention, « un » doit se comprendre, sauf indication contraire, au sens de « au moins un ».

Les exemples et figures ci-après sont présentés à titre illustratif et non limitatif de l'invention.

### EXEMPLES

### Exemple 1

### Expression de l'IDE dans le cuir chevelu

### 1- Matériels et méthodes

Une analyse protéomique différentielle par approche 2D-électrophorèse effectuée à partir de prélèvements non-invasifs de scalps non pelliculaires et pelliculaires a été réalisée.

L'étude est effectuée sur 6 volontaires non pelliculaires (grade pellicules adhérentes de 0 à 0,25) et 6 volontaires pelliculaires (grade pellicules adhérentes de 3,25 à 4). Les volontaires sont des hommes aux cheveux courts de 36 à 39 ans. La gradation des états pelliculaires des volontaires est effectuée par un expert selon la classification standard suivante ou prévoit une échelle de score des pellicules adhérentes comprise entre 0 et 5.

Les prélèvements sont effectués par cornéodisque (référence GODS100, CuDerm) sur 1 zone de 2cm. Quatre cornéodisques à saturation sont réalisés pour chaque prélèvement. Les protéines solubles sont extraites dans un tampon natif (TBS, 1M NaCl, 1 % triton X100).

Après filtration, les protéines sont précipitées par addition d'acétone. Le culot protéique est dissout dans le tampon III d'extraction (BioRad ; Réf: 163-2104) complémenté avec 40 mM de DTT.

Pour chaque échantillon correspondant à un volontaire, une électrophorèse bidimensionnelle est réalisée selon une première dimension par séparation à IEF pH 3-11 sur, strip de 11 cm (GE-Healthcare) et selon une seconde dimension sur un gel gradient Critérien 10,5-14 % Bio-Rad) selon les recommandations des fournisseurs.

Après coloration des gels avec du SyproRuby (référence S4942, Invitrogen) utilisé selon le protocole du fournisseur; une analyse d'image est effectuée avec le logiciel PROGENESIS™ après acquisition des images selon les paramètres suivants : Excitation : 460/80 ; Emission : 620/30 ; Résolution 50µm ; Exposition 3 s ; Flat field : 1 s ; Exposition 1.

Dans une première étape, les images sont alignées ensemble et les intensités normalisées. Dans une seconde étape, après avoir défini des groupes, une analyse statistique est réalisée par le logiciel Progenesis Samespots (NonLinear Dynamics). Une sélection des spots est effectuée en fonction de la « p value » (inférieure à 0,05), le « fold » (supérieur à 2, ratio d'intensité entre le spot le plus intense d'un groupe et le spot le moins intense d'un autre groupe) et la « q value » (supérieure à 0,8).

Les spots sélectionnés sont ensuite découpés, les protéines sont digérées par la trypsine et analysées par LC-MS/MS.

La banque protéique UniRef100.15.3.9606.homo-sapiens a été interrogée pour identifier les protéines.

### 2- Résultats

L'IDE est identifiée parmi les spots sélectionnés en faveur d'une surexpression dans le groupe pelliculaire selon les critères définis précédemment.

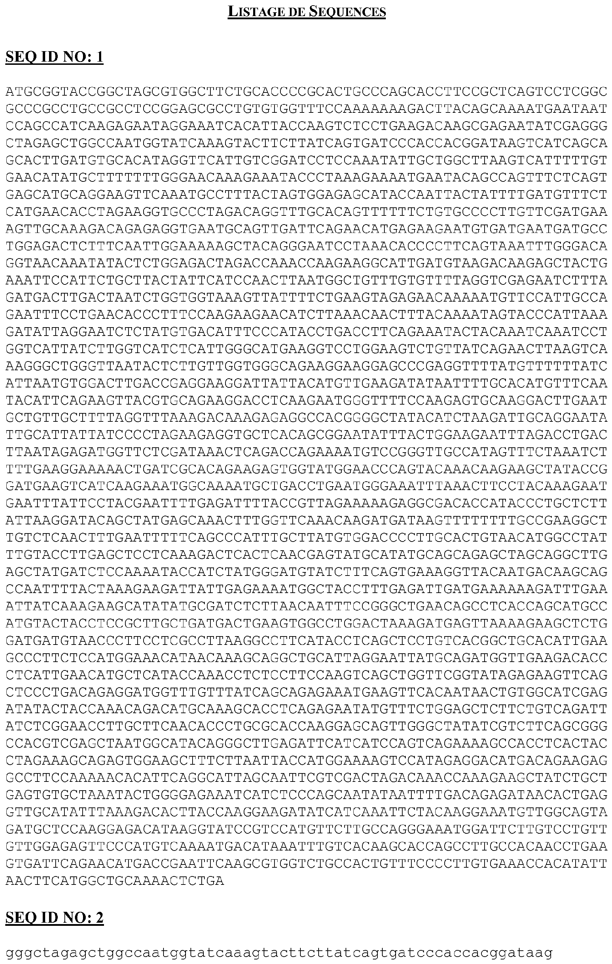

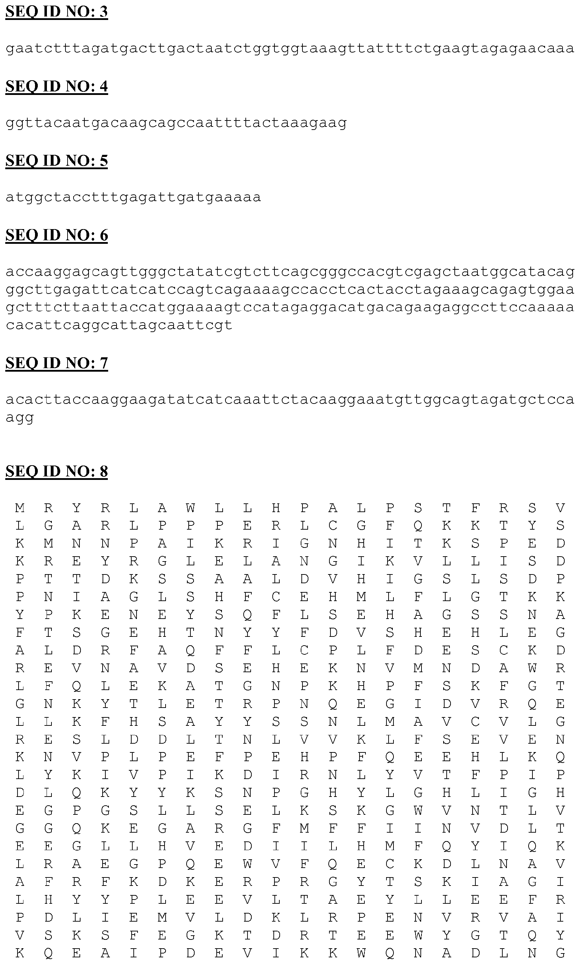

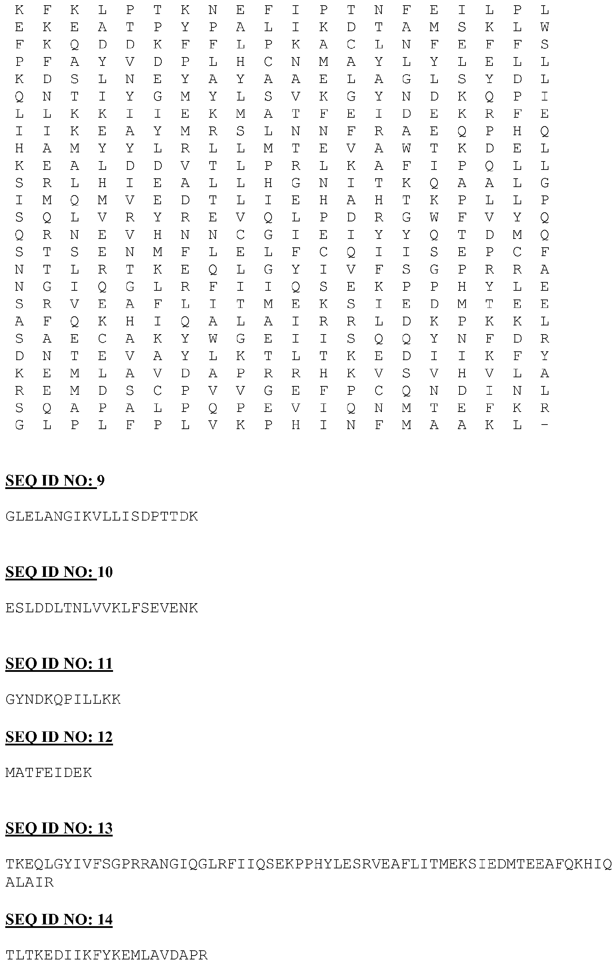

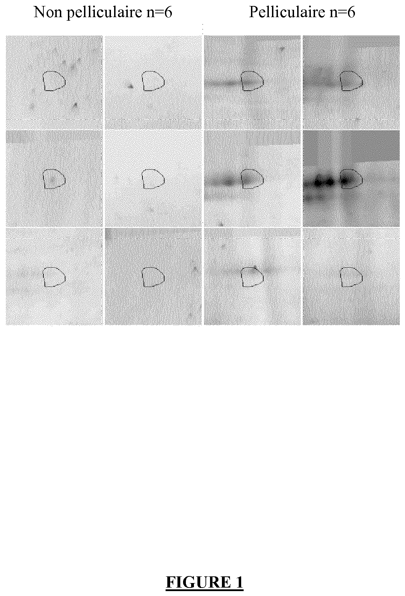

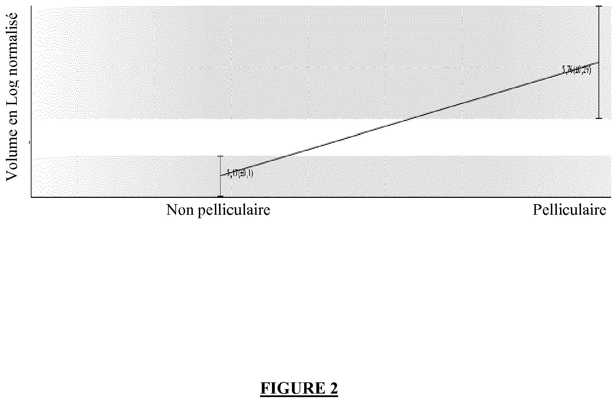

L'agrandissement de ce spot protéique pour chaque volontaire ainsi que la représentation graphique du volume des spots est donné par les Figures 1 et 2.

Ces résultats valident l'utilisation de l'IDE comme biomarqueur de la peau, en particulier de la desquamation de la peau, et plus particulièrement d'un état pelliculaire du cuir chevelu. L'IDE peut ainsi être avantageusement utilisée pour le criblage d'actifs pour le traitement d'un état pelliculaire du cuir chevelu ou pour caractériser l'efficacité d'un traitement cosmétique de la peau.

### Exemple 2

### Confirmation par Western blot de l'expression de l'IDE dans la peau

### 1- Matériels et méthodes

La concentration des échantillons précédemment décrits dans l'exemple 1 est alignée, et 2 µg sont déposés dans les puits d'un gel Critérion 10-20 % (BioRad). Les protéines sont ainsi séparées par électrophorèse SDS-PAGE. Après transfert semi-sec sur une membrane de PVDF selon un protocole standard, les protéines sont incubées avec un anticorps primaire dirigé contre la protéine d'intérêt toute la nuit à 4 °C. Une seconde incubation est ensuite réalisée avec un anticorps secondaire (couplé à une peroxydase) dirigé contre le premier anticorps.

Un kit d'électro-chemiluminescence est utilisé pour la révélation des protéines ciblées.

L'image est acquise au FluorSmax (Biorad) et les bandes quantifiées à l'aide du logiciel Quantity-One (Biorad). Un anticorps commercial anti-IDE a été utilisé à la dilution de 1/5000eme (référence AB28560, AbCam)

### 2- Résultats

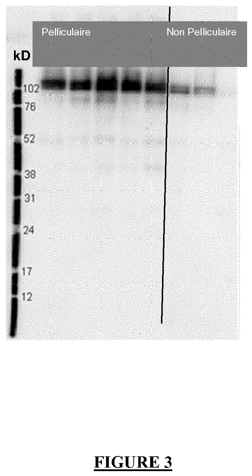

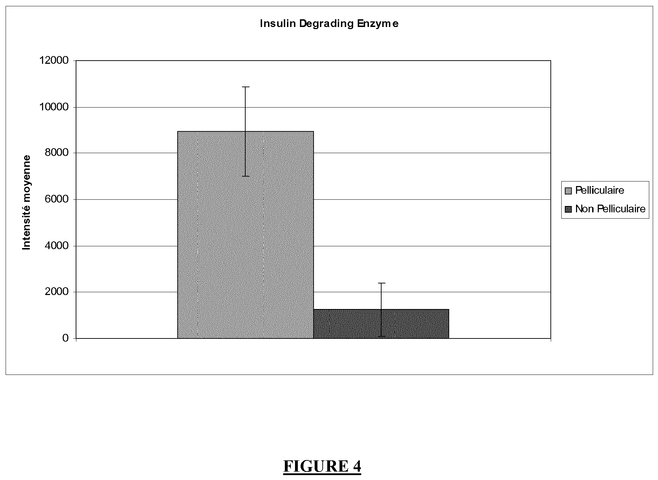

La Figure 3 met en évidence l'image du Western Blot obtenue. L'intensité moyenne des bandes détectées est représentée par l'histogramme en Figure 4.

L'immunodétection de l'IDE met en évidence une surexpression chez les sujets pelliculaires. Cette observation confirme les identifications différentielles observées dans l'exemple 1.

### BIBLIOGRAPHIE

Ali et al. (2009). "The insulin degrading enzyme binding domain of varicella-zoster virus (VZV) glycoprotein E is important for cell-to-cell spread and VZV infectivity, while a glycoprotein I binding domain is essential for infection." Virology 386(2): 270-279.
Cabrol et al. (2009). "Small-molecule activators of insulin-degrading enzyme discovered through high-throughput compound screening." PLoS One 4(4): e5274.
Camberos et al. (2001). "ATP inhibits insulin-degrading enzyme activity." Exp Biol Med (Maywood) 226(4): 334-341.
Groves et al. (2003). "Association and haplotype analysis of the insulin-degrading enzyme (IDE) gene, a strong positional and biological candidate for type 2 diabetes susceptibility." Diabetes 52(5): 1300-1305.
Guo et al. (2010). "Molecular Basis for the Récognition and Cleavages of IGF-II, TGF-alpha, and Amylin by Human Insulin-Degrading Enzyme." J Mol Biol 395(2) : 430-443.
Kim et al. (2007). "Decreased catalytic activity of the insulin-degrading enzyme in chromosome 10-linked Alzheimer disease families." J Biol Chem 282(11): 7825-7832.
Miners et al. (2009). "Neprilysin and insulin-degrading enzyme levels are increased in Alzheimer disease in relation to disease severity." J Neuropathol Exp Neurol 68(8): 902-914.
Radulescu et al. (2007). "Immunohistochemical démonstration of the zinc metalloprotease insulin-degrading enzyme in normal and malignant human breast: Correlation with tissue insulin levels." Int J Oncol 30:73-80.
Shearer et al. (1997). "Insulin is degraded extracellularly in wounds by insulin-degrading enzyme (EC 3.4.24.56)." Am J Physiol 273(4 Pt 1): E657-664.
Kyte et al. (1982), J Mol Biol, 157: 105.
Mehul et al. (2000) "Identification and Cloning of a New Calmodulin-like Protein from Human Epidermis" J Biol Chem 275(17): 12841-12847
Sambrook *et al.* (1989), Vol. I-III, Coldspring Harbor Laboratory, Coldspring Harbor Press, NY.
« Antibodies: A Laboratory Manual », Cold Spring Harbor Laboratory Press, Cold Spring Harbor, N.Y. (1990).

## Revendications

1. Utilisation *in vitro* ou *ex vivo* (i) d'au moins une séquence d'acides aminés codée par une séquence d'acides nucléiques SEQ ID NO: 1, un analogue ou un fragment de SEQ ID NO: 1, ledit analogue ayant une identité de séquence d'au moins 85 % avec la séquence SEQ ID NO : 1 et codant pour une séquence d'acides aminés ayant une activité biologique de même nature que la séquence d'acides aminés codée par la séquence SEQ ID NO : 1, ledit fragment comprenant de 9 à 300 paires de bases consécutives de la séquence SEQ ID NO : 1 et codant pour une séquence d'acides aminés ayant une activité biologique de même nature que la séquence d'acides aminés codée par la séquence SEQ ID NO : 1, ou (ii) d'au moins de ladite séquence d'acides nucléiques, à titre de biomarqueur d'un état pelliculaire du cuir chevelu.

2. Utilisation selon la revendication précédente, dans laquelle ladite séquence d'acides nucléiques est choisie parmi SEQ ID NO: 2 à SEQ ID NO: 7, ou un analogue de celle-ci, ledit analogue ayant une identité de séquence d'au moins 85 % avec ladite séquence d'acides nucléiques et codant pour une séquence d'acides aminés ayant une activité biologique de même nature que la séquence d'acides aminés codée par ladite séquence d'acides nucléiques.

3. Utilisation selon la revendication 1 ou 2, dans laquelle ladite séquence d'acides aminés est choisie parmi SEQ ID NO: 8 à SEQ ID NO: 14, ou un analogue de celle-ci, ledit analogue ayant une identité de séquence d'au moins 85 % avec ladite séquence d'acides aminés et ayant une activité biologique de même nature que ladite séquence d'acides aminés.

4. Utilisation *in vitro* ou *ex vivo* (i) d'au moins une séquence d'acides aminés telle que définie en revendications 1 à 3, ou (ii) d'au moins une séquence d'acides nucléiques telle que définie en revendications 1 ou 2, pour caractériser l'efficacité d'un traitement cosmétique d'un état pelliculaire du cuir chevelu.

5. Utilisation *in vitro* ou *ex vivo* (i) d'au moins une séquence d'acides aminés telle que définie en revendications 1 à 3, ou (ii) d'au moins une séquence d'acides nucléiques telle que définie en revendications 1 ou 2, pour sélectionner parmi un ensemble d'agents actifs connus pour prévenir et/ou traiter un état pelliculaire du cuir chevelu, un agent actif présumé exercer un effet bénéfique maximal à l'égard dudit état pelliculaire.

6. Procédé *in vitro* ou *ex vivo* pour caractériser un état pelliculaire du cuir chevelu comprenant au moins les étapes consistant à :
a) effectuer, dans un échantillon isolé d'un cuir chevelu, une mesure de l'expression, de la maturation ou de l'activité d'une séquence d'acides aminés telle que définie en revendications 1 à 3, et
b) comparer ladite mesure effectuée à l'étape a) à une mesure de référence ;
une augmentation de l'activité, de l'expression ou de la maturation de ladite séquence d'acides aminées étant indicative d'un état pelliculaire du cuir chevelu.

7. Procédé cosmétique, *in vitro* ou *ex vivo,* pour caractériser l'efficacité d'un traitement cosmétique d'un état pelliculaire du cuir chevelu chez un individu en ayant besoin, comprenant au moins les étapes consistant à :
a) effectuer, avant la mise en oeuvre du traitement cosmétique, dans un premier échantillon de cuir chevelu isolé prélevé chez ledit individu, au moins une première mesure de l'expression, de la maturation ou de l'activité d'au moins une séquence d'acides aminés telle que définie en revendications 1 à 3, ou d'au moins une séquence d'acides nucléiques telle que définie en revendications 1 ou 2,
b) effectuer, après la mise en oeuvre du traitement cosmétique, dans un second échantillon de cuir chevelu isolé prélevé chez ledit individu, au moins une seconde mesure, qualitative ou quantitative de l'expression, de la maturation ou de l'activité de ladite séquence d'acides aminés ou de ladite séquence d'acides nucléiques, et
c) comparer les première et deuxième mesures, notamment afin d'en déduire une information relative à un effet au moins de la mise en oeuvre du traitement cosmétique.

8. Peptide isolé choisi parmi SEQ ID NO: 9 à SEQ ID NO: 14, ou un analogue ayant une identité de séquence d'au moins 85 % avec ledit peptide isolé et ayant une activité biologique de même nature que ledit peptide isolé.

9. Composition comprenant un peptide tel que défini selon la revendication précédente ou une séquence d'acides nucléiques codant pour un tel peptide.

## Patentansprüche

1. Verwendung, in vitro oder ex vivo (i) von mindestens einer Aminosäurensequenz, die von einer Nukleinsäurensequenz SEQ ID NO: 1, einem Analog oder einem Fragment von SEQ ID NO: 1 kodiert wird, wobei das Analog eine Sequenzidentiät von mindestens 85 % mit der Sequenz SEQ ID NO: 1 aufweist und eine Aminosäurensequenz mit einer biologischen Aktivität der gleichen Art wie die von der Sequenz SEQ ID NO: 1 kodierte Aminosäurensequenz kodiert, wobei das Fragment von 9 bis 300 aufeinanderfolgende Basenpaare der Sequenz SEQ ID NO: 1 umfasst und eine Aminosäurensequenz mit einer biologischen Aktivität der gleichen Art wie die von der Sequenz SEQ ID NO: 1 kodierte Aminosäurensequenz kodiert, oder (ii) von mindestens der Nukleinsäurensequenz, als Biomarker einer Kopfhauterkrankung.

2. Verwendung nach dem vorangehenden Anspruch, wobei die Nukleinsäurensequenz ausgewählt ist aus SEQ ID NO: 2 bis SEQ ID NO: 7, oder einem Analog davon, wobei das Analog eine Sequenzidentiät von mindestens 85 % mit der Nukleinsäurensequenz aufweist und eine Aminosäurensequenz mit einer biologischen Aktivität der gleichen Art wie die von der Nukleinsäurensequenz kodierte Aminosäurensequenz kodiert.

3. Verwendung nach Anspruch 1 oder 2, wobei die Aminosäurensequenz ausgewählt ist aus SEQ ID NO: 8 bis SEQ ID NO: 14, oder einem Analog davon, wobei das Analog eine Sequenzidentiät von mindestens 85 % mit der Aminosäurensequenz aufweist und mit einer biologischen Aktivität der gleichen Art wie die Aminosäurensequenz.

4. Verwendung, in vitro oder ex vivo, (i) von mindestens einer Aminosäurensequenz nach einem der Ansprüche 1 bis 3, oder (ii) von mindestens einer Nukleinsäurensequenz nach den Ansprüchen 1 oder 2, zur Charakterisierung der Wirksamkeit einer kosmetischen Behandlung einer Kopfhauterkrankung.

5. Verwendung, in vitro oder ex vivo, (i) von mindestens einer Aminosäurensequenz nach den Ansprüchen 1 bis 3, oder (ii) von mindestens einer Nukleinsäurensequenz nach den Ansprüchen 1 oder 2, zur Selektion aus einer Gesamtheit von Wirkstoffen, die bekanntermaßen eine Kopfhauterkrankung verhindern und/oder behandeln, eines Wirkstoffs, der geschätzt eine besonders gesundheitsfördernde Wirkung hinsichtlich der Kopfhauterkrankung ausübt.

6. In-vitro- oder Ex-vivo-Verfahren zur Charakterisierung einer Kopfhauterkrankung, umfassend mindestens die folgenden Schritte:
a) Durchführens in einer isolierte Probe einer Kopfhaut einer Messung der Expression, der Maturation oder der Aktivität einer Aminosäurensequenz nach den Ansprüchen 1 bis 3, und
b) Vergleichen der in Schritt a) durchgeführten Messung mit einer Referenzmessung;
wobei eine Steigerung der Aktivität, der Expression oder der Maturation der Aminosäurensequenz ein Hinweis ist auf eine Kopfhauterkrankung.

7. Kosmetisches In-vitro- oder Ex-vivo-Verfahren zur Charakterisierung der Wirksamkeit einer kosmetischen Behandlung einer Kopfhauterkrankung bei einem Individuum, das Bedarf daran hat, umfassend mindestens die folgenden Schritte:
a) Durchführen, vor der Durchführung der kosmetischen Behandlung, in einer ersten Probe der von einem Individuum entnommenen isolierten Kopfhaut, mindestens einer ersten Messung der Expression, der Maturation oder der Aktivität von mindestens einer Aminosäurensequenz nach den Ansprüchen1 bis 3, oder von mindestens einer Nukleinsäurensequenz nach den Ansprüchen 1 oder 2,
b) Durchführen, nach der Durchführung der kosmetischen Behandlung, in einer zweiten Probe der von einem Individuum entnommenen isolierten Kopfhaut, mindestens einer zweiten qualitativen oder quantitativen Messung, der Expression, der Maturation oder der Aktivität der Aminosäurensequenz oder der Nukleinsäurensequenz, und
c) Vergleichen der ersten und der zweiten Messungen, insbesondere um eine Information bezüglich einer Wirkung von mindestens der Durchführung der kosmetischen Behandlung abzuleiten.

8. Isoliertes Peptid, ausgewählt aus SEQ ID NO: 9 bis SEQ ID NO: 14, oder einem Analog mit einer Sequenzidentiät von mindestens 85 % mit dem isolierten Peptide und mit einer biologischen Aktivität der gleichen Art wie das isolierte Peptid.

9. Zusammensetzung, umfassend ein Peptid wie im vorangehenden Anspruch definiert, oder eine Nukleinsäurensequenz, die ein solches Peptid kodiert.

## Claims

1. Use *in vitro or ex vivo* (i) of at least one amino acid sequence encoded by a sequence of nucleic acid SEQ ID NO 1, an analog or a fragment of SEQ ID NO 1, wherein the said analog has a sequence identity of at least 85% with the sequence SEQ ID NO 1, and encoding for a sequence of amino acids having a biological activity of the same nature as the amino acid sequence encoded by the sequence SEQ ID NO 1, wherein the said fragment comprises from 9 to 300 consecutive base pairs of the sequence SEQ ID NO 1 and encoding for an amino acid sequence having a biological activity of the same nature as the amino acid sequence encoded by the sequence SEQ ID NO 1, or (ii) of at least one of the said nucleic acid sequence, as a biomarker of a dandruff condition of the scalp.

2. Use according to the preceding claim, wherein the said nucleic acid sequence is selected from among SEQ ID NO 2 to SEQ ID NO 7, or an analog thereof, wherein the said analog has a sequence identity of at least 85% with the said nucleic acid sequence, and encoding for an amino acid sequence having a biological activity of the same nature as the amino acid sequence encoded by the said nucleic acid sequence.

3. Use according to claim 1 or 2, wherein the said amino acid sequence is selected from among SEQ ID NO 8 to SEQ ID NO 14, or an analog thereof, wherein the said analog has a sequence identity of at least 85% with the said amino acid sequence, and has a biological activity of the same nature as the said amino acid sequence.

4. Use *in vitro or ex vivo* (i) of at least one amino acid sequence as defined in claims 1 to 3, or (ii) of at least one nucleic acid sequence as defined in claims 1 or 2, to characterize the effectiveness of a cosmetic treatment of a dandruff condition of the scalp.

5. Use *in vitro or ex vivo* (i) of at least one amino acid sequence as defined in claims 1 to 3, or (ii) of at least one nucleic acid sequence as defined in claims 1 or 2, in order to select from a set of active agents known to prevent and/or treat a dandruff condition of the scalp, wherein an active agent is presumed to exert a maximum beneficial effect with respect to the said dandruff condition.

6. Method *in vitro or ex* vivo for characterizing a scalp dandruff condition comprising at least the steps of:
a) performing, on an isolated sample of a scalp, a measurement of the expression, the maturation or the activity of an amino acid sequence as defined in claims 1 to 3, and
(b) comparing the measurement made in step (a) with a reference measurement;
wherein an increase in the activity, expression or maturation of the said amino acid sequence is indicative of a dandruff condition of the scalp.

7. Cosmetic process, *in vitro or ex vivo,* for characterizing the efficacy of a cosmetic treatment of a scalp condition of the scalp in an individual requiring it, comprising at least the steps of:
a) performing, before the implementation of the cosmetic treatment, on a first isolated scalp sample taken from the said individual, at least a first measurement of the expression, the maturation or the activity of at least one amino acid sequence as defined in claims 1 to 3, or at least one nucleic acid sequence as defined in claims 1 or 2,
b) performing, after the implementation of the cosmetic treatment, in a second isolated scalp sample from the said individual, at least a second, qualitative or quantitative measurement of the expression, the maturation or the activity of the said amino acid sequence or the said nucleic acid sequence, and
c) comparing the first and second measurements, in particular to derive information relating to at least one effect of the implementation of the cosmetic treatment.

8. Isolated peptide selected from among SEQ ID NO 9 to SEQ ID NO 14, or an analog having a sequence identity of at least 85% with the said isolated peptide, and having a biological activity of the same nature as the said isolated peptide.

9. Composition comprising a peptide such as that defined according to the preceding claim or a sequence of encoding nucleic acids for such a peptide.


### SEQUENCE LISTING

<110> L'OREAL
<120> Utilisation de l'IDE comme biomarqueur dâ\200\231un Ã©tat du cuir chevelu
<130> PR94529
<150> FR1060429
   <151> 2010-12-13
   <150> US61457083
   <151> 2010-12-23
<160> 14
<170> BiSSAP 1.0
<210> 1
   <211> 3060
   <212> DNA
   <213> Homo sapiens
<220>
   <221> source
   <222> 1..3060
   <223> /mol_type="DNA"
   /organism="Homo sapiens"
<400> 1
   atgcggtacc ggctagcgtg gcttctgcac cccgcactgc ccagcacctt ccgctcagtc      60
   ctcggcgccc gcctgccgcc tccggagcgc ctgtgtggtt tccaaaaaaa gacttacagc     120
   aaaatgaata atccagccat caagagaata ggaaatcaca ttaccaagtc tcctgaagac     180
   aagcgagaat atcgagggct agagctggcc aatggtatca aagtacttct tatcagtgat     240
   cccaccacgg ataagtcatc agcagcactt gatgtgcaca taggttcatt gtcggatcct     300
   ccaaatattg ctggcttaag tcatttttgt gaacatatgc tttttttggg aacaaagaaa     360
   taccctaaag aaaatgaata cagccagttt ctcagtgagc atgcaggaag ttcaaatgcc     420
   tttactagtg gagagcatac caattactat tttgatgttt ctcatgaaca cctagaaggt     480
   gccctagaca ggtttgcaca gttttttctg tgccccttgt tcgatgaaag ttgcaaagac     540
   agagaggtga atgcagttga ttcagaacat gagaagaatg tgatgaatga tgcctggaga     600
   ctctttcaat tggaaaaagc tacagggaat cctaaacacc ccttcagtaa atttgggaca     660
   ggtaacaaat atactctgga gactagacca aaccaagaag gcattgatgt aagacaagag     720
   ctactgaaat tccattctgc ttactattca tccaacttaa tggctgtttg tgttttaggt     780
   cgagaatctt tagatgactt gactaatctg gtggtaaagt tattttctga agtagagaac     840
   aaaaatgttc cattgccaga atttcctgaa caccctttcc aagaagaaca tcttaaacaa     900
   ctttacaaaa tagtacccat taaagatatt aggaatctct atgtgacatt tcccatacct     960
   gaccttcaga aatactacaa atcaaatcct ggtcattatc ttggtcatct cattgggcat    1020
   gaaggtcctg gaagtctgtt atcagaactt aagtcaaagg gctgggttaa tactcttgtt 1080
   ggtgggcaga aggaaggagc ccgaggtttt atgtttttta tcattaatgt ggacttgacc 1140
   gaggaaggat tattacatgt tgaagatata attttgcaca tgtttcaata cattcagaag 1200
   ttacgtgcag aaggacctca agaatgggtt ttccaagagt gcaaggactt gaatgctgtt 1260
   gcttttaggt ttaaagacaa agagaggcca cggggctata catctaagat tgcaggaata 1320
   ttgcattatt atcccctaga agaggtgctc acagcggaat atttactgga agaatttaga 1380
   cctgacttaa tagagatggt tctcgataaa ctcagaccag aaaatgtccg ggttgccata 1440
   gtttctaaat cttttgaagg aaaaactgat cgcacagaag agtggtatgg aacccagtac 1500
   aaacaagaag ctataccgga tgaagtcatc aagaaatggc aaaatgctga cctgaatggg 1560
   aaatttaaac ttcctacaaa gaatgaattt attcctacga attttgagat tttaccgtta 1620
   gaaaaagagg cgacaccata ccctgctctt attaaggata cagctatgag caaactttgg 1680
   ttcaaacaag atgataagtt ttttttgccg aaggcttgtc tcaactttga atttttcagc 1740
   ccatttgctt atgtggaccc cttgcactgt aacatggcct atttgtacct tgagctcctc 1800
   aaagactcac tcaacgagta tgcatatgca gcagagctag caggcttgag ctatgatctc 1860
   caaaatacca tctatgggat gtatctttca gtgaaaggtt acaatgacaa gcagccaatt 1920
   ttactaaaga agattattga gaaaatggct acctttgaga ttgatgaaaa aagatttgaa 1980
   attatcaaag aagcatatat gcgatctctt aacaatttcc gggctgaaca gcctcaccag 2040
   catgccatgt actacctccg cttgctgatg actgaagtgg cctggactaa agatgagtta 2100
   aaagaagctc tggatgatgt aacccttcct cgccttaagg ccttcatacc tcagctcctg 2160
   tcacggctgc acattgaagc ccttctccat ggaaacataa caaagcaggc tgcattagga 2220
   attatgcaga tggttgaaga caccctcatt gaacatgctc ataccaaacc tctccttcca 2280
   agtcagctgg ttcggtatag agaagttcag ctccctgaca gaggatggtt tgtttatcag 2340
   cagagaaatg aagttcacaa taactgtggc atcgagatat actaccaaac agacatgcaa 2400
   agcacctcag agaatatgtt tctggagctc ttctgtcaga ttatctcgga accttgcttc 2460
   aacaccctgc gcaccaagga gcagttgggc tatatcgtct tcagcgggcc acgtcgagct 2520
   aatggcatac agggcttgag attcatcatc cagtcagaaa agccacctca ctacctagaa 2580
   agcagagtgg aagctttctt aattaccatg gaaaagtcca tagaggacat gacagaagag 2640
   gccttccaaa aacacattca ggcattagca attcgtcgac tagacaaacc aaagaagcta 2700
   tctgctgagt gtgctaaata ctggggagaa atcatctccc agcaatataa ttttgacaga 2760
   gataacactg aggttgcata tttaaagaca cttaccaagg aagatatcat caaattctac 2820
   aaggaaatgt tggcagtaga tgctccaagg agacataagg tatccgtcca tgttcttgcc 2880
   agggaaatgg attcttgtcc tgttgttgga gagttcccat gtcaaaatga cataaatttg 2940
   tcacaagcac cagccttgcc acaacctgaa gtgattcaga acatgaccga attcaagcgt 3000
   ggtctgccac tgtttcccct tgtgaaacca catattaact tcatggctgc aaaactctga 3060
<210> 2
   <211> 60
   <212> DNA
   <213> Homo sapiens
<220>
   <221> source
   <222> 1..60
   <223> /mol_type="DNA"
   /organism="Homo sapiens"
<400> 2
   gggctagagc tggccaatgg tatcaaagta cttcttatca gtgatcccac cacggataag 60
<210> 3
   <211> 60
   <212> DNA
   <213> Homo sapiens
<220>
   <221> source
   <222> 1..60
   <223> /mol_type="DNA" /organism="Homo sapiens"
<400> 3
   gaatctttag atgacttgac taatctggtg gtaaagttat tttctgaagt agagaacaaa 60
<210> 4
   <211> 36
   <212> DNA
   <213> Homo sapiens
<220>
   <221> source
   <222> 1..36
   <223> /mol_type="DNA" /organism="Homo sapiens"
<400> 4
   ggttacaatg acaagcagcc aattttacta aagaag 36
<210> 5
   <211> 27
   <212> DNA
   <213> Homo sapiens
<220>
   <221> source
   <222> 1..27
   <223> /mol_type="DNA"
   /organism="Homo sapiens"
<400> 5
   atggctacct ttgagattga tgaaaaa 27
<210> 6
   <211> 204
   <212> DNA
   <213> Homo sapiens
<220>
   <221> source
   <222> 1..204
   <223> /mol_type="DNA"
   /organism="Homo sapiens"
<400> 6
   accaaggagc agttgggcta tatcgtcttc agcgggccac gtcgagctaa tggcatacag      60
   ggcttgagat tcatcatcca gtcagaaaag ccacctcact acctagaaag cagagtggaa     120
   gctttcttaa ttaccatgga aaagtccata gaggacatga cagaagaggc cttccaaaaa     180
   cacattcagg cattagcaat tcgt                                            204
<210> 7
   <211> 63
   <212> DNA
   <213> Homo sapiens
<220>
   <221> source
   <222> 1..63
   <223> /mol_type="DNA" /organism="Homo sapiens"
<400> 7
   acacttacca aggaagatat catcaaattc tacaaggaaa tgttggcagt agatgctcca      60
   agg                                                                    63
<210> 8
   <211> 1019
   <212> PRT
   <213> Homo sapiens
<220>
   <221> SOURCE
   <222> 1..1019
   <223> /mol_type="protein"
   /organism="Homo sapiens"
<400> 8
<210> 9
   <211> 20
   <212> PRT
   <213> Homo sapiens
<220>
   <221> SOURCE
   <222> 1..20
   <223> /mol_type="protein"
   /organism="Homo sapiens"
<400> 9
   Gly Leu Glu Leu Ala Asn Gly Ile Lys Val Leu Leu Ile Ser Asp Pro
   1               5                   10                  15
   Thr Thr Asp Lys
               20
<210> 10
   <211> 20
   <212> PRT
   <213> Homo sapiens
<220>
   <221> SOURCE
   <222> 1..20
   <223> /mol_type="protein"
   /organism="Homo sapiens"
<400> 10
   Glu Ser Leu Asp Asp Leu Thr Asn Leu Val Val Lys Leu Phe Ser Glu
   1               5                   10                  15
   Val Glu Asn Lys
               20
<210> 11
   <211> 12
   <212> PRT
   <213> Homo sapiens
<220>
   <221> SOURCE
   <222> 1..12
   <223> /mol_type="protein"
   /organism="Homo sapiens"
<400> 11
   Gly Tyr Asn Asp Lys Gln Pro Ile Leu Leu Lys Lys
   1               5                   10
<210> 12
   <211> 9
   <212> PRT
   <213> Homo sapiens
<220>
   <221> SOURCE
   <222> 1..9
   <223> /mol_type="protein"
   /organism="Homo sapiens"
<400> 12
   Met Ala Thr Phe Glu Ile Asp Glu Lys
   1               5
<210> 13
   <211> 68
   <212> PRT
   <213> Homo sapiens
<220>
   <221> SOURCE
   <222> 1..68
   <223> /mol_type="protein"
   /organism="Homo sapiens"
<400> 13
   Thr Lys Glu Gln Leu Gly Tyr Ile Val Phe Ser Gly Pro Arg Arg Ala
   1               5                   10                  15
   Asn Gly Ile Gln Gly Leu Arg Phe Ile Ile Gln Ser Glu Lys Pro Pro
               20                  25                  30
   His Tyr Leu Glu Ser Arg Val Glu Ala Phe Leu Ile Thr Met Glu Lys
           35                  40                  45
   Ser Ile Glu Asp Met Thr Glu Glu Ala Phe Gln Lys His Ile Gln Ala
       50                  55                  60
   Leu Ala Ile Arg
   65
<210> 14
   <211> 21
   <212> PRT
   <213> Homo sapiens
<220>
   <221> SOURCE
   <222> 1..21
   <223> /mol_type="protein"
   /organism="Homo sapiens"
<400> 14
   Thr Leu Thr Lys Glu Asp Ile Ile Lys Phe Tyr Lys Glu Met Leu Ala
   1               5                   10                  15
   Val Asp Ala Pro Arg
               20